# EUROPEAN PATENT APPLICATION

(11) **EP 4 513 510 A1**
(43) Date of publication of application: **26.02.2025**
(21) Application number: 24792017.6
(22) Date of filing: 17.04.2024
(51) Int. Cl.: G16H 50/30, G16H 40/63, G16H 10/60, A61B 5/145

(54) **BLOOD GLUCOSE ASSESSMENT METHOD, ELECTRONIC DEVICE, AND COMPUTER READABLE STORAGE MEDIUM**

(30) Priority: 18.04.2023 CN 202310461224
(71) Applicant: Huawei Technologies Co., Ltd., Shenzhen, Guangdong 518129 (CN); Peking Union Medical College Hospital, Chinese Academy of Medical Sciences, Dongcheng District Beijing 100730 (CN)
(72) Inventor: LI, Liangzhi, Shenzhen, Guangdong 518129 (CN); CHEN, Maolin, Shenzhen, Guangdong 518129 (CN); LI, Luping, Shenzhen, Guangdong 518129 (CN); ZHOU, Hui, Shenzhen, Guangdong 518129 (CN); JIA, Ziqian, Shenzhen, Guangdong 518129 (CN); ZHANG, Xuexing, Shenzhen, Guangdong 518129 (CN)
(74) Representative: Körber, Martin Hans
(86) International application number: PCT/CN2024/088170
(87) International publication number: WO 2024/217432

(57) **Abstract**

This application provides a blood glucose assessment method, an electronic device, and a computer-readable storage medium. The method is applied to a wearable device, and includes: collecting vital sign data of a user in a first cycle of blood glucose assessment; performing blood glucose assessment on the user based on valid vital sign data in the vital sign data, to obtain a blood glucose assessment result corresponding to the first cycle; displaying the blood glucose assessment result corresponding to the first cycle; and automatically starting a next cycle of the blood glucose assessment. According to the technical solutions of this application, blood glucose assessment can be periodically and automatically performed, helping reduce operation complexity for the user.

## Description

This application claims priority to Chinese Patent Application No. 202310461224.1, filed with the China National Intellectual Property Administration on April 18, 2023, and entitled "BLOOD GLUCOSE ASSESSMENT METHOD, ELECTRONIC DEVICE, AND COMPUTER-READABLE STORAGE MEDIUM", which is incorporated herein by reference in its entirety.

### TECHNICAL FIELD

This application relates to the field of smart wearable device technologies, and in particular, to a blood glucose assessment method, an electronic device, and a computer-readable storage medium.

### BACKGROUND

Diabetes is a disease with excessive glucose accumulation in blood, and has features of high morbidity and great damage to human health. Because diabetic or pre-diabetic patients do not have special symptoms in an early stage, the patients have a low awareness rate of the diabetes or pre-diabetes, and it is likely to miss best time for diagnosis and treatment. For example, among one billion adults, a pre-diabetic population has a prevalence rate of 35.2%, but an awareness rate of only 20%, and a type 2 diabetic population has a prevalence rate of 12.8%, but an awareness rate of only 43.3%.

Currently, human blood glucose may be assessed in an invasive manner or a non-invasive manner, to determine a diabetic or pre-diabetic risk of a user. The non-invasive manner may be implemented based on a questionnaire screening manner, and is a one-time monitoring manner. In addition, the user needs to actively enter a large amount of related information, and operation complexity for the user is high.

### SUMMARY

Embodiments of this application provide a blood glucose assessment method, an electronic device, and a computer-readable storage medium, to periodically and automatically perform blood glucose assessment, helping reduce operation complexity for a user.

According to a first aspect, an embodiment of this application provides a blood glucose assessment method, which may be applied to a wearable device. The method includes: collecting vital sign data of a user in a first cycle of blood glucose assessment; performing blood glucose assessment on the user based on valid vital sign data in the vital sign data, to obtain a blood glucose assessment result corresponding to the first cycle; displaying the blood glucose assessment result corresponding to the first cycle; and automatically starting a next cycle of the blood glucose assessment.

According to the method described in the first aspect, in a blood glucose assessment process, the blood glucose assessment result corresponding to the first cycle may be first obtained, and then the next cycle is automatically started, to obtain a blood glucose assessment result corresponding to the next cycle. Based on this, blood glucose assessment may be periodically and automatically performed on the user. In addition, in the first cycle, the vital sign data of the user may be collected, and blood glucose assessment may be performed on the user based on the valid vital sign data in the vital sign data. In this process, the user does not need to actively enter a large amount of information, helping reduce operation complexity for the user.

With reference to the first aspect, in a possible implementation, the vital sign data includes first vital sign data collected in a first time period and second vital sign data collected in a second time period, the first time period and the second time period are two time periods on a same day, and the method further includes: determining first cumulative total duration and second cumulative total duration, where the first cumulative total duration is cumulative total duration for collecting first valid vital sign data in the first cycle, and the second cumulative total duration is cumulative total duration for collecting second valid vital sign data in the first cycle, the first valid vital sign data is valid vital sign data in the first vital sign data, and the second valid vital sign data is valid vital sign data in the second vital sign data. The performing blood glucose assessment on the user based on valid vital sign data in the vital sign data includes: if the first cumulative total duration is greater than a first threshold, and the second cumulative total duration is greater than a second threshold, performing blood glucose assessment on the user based on the first valid vital sign data in the first vital sign data and the second valid vital sign data in the second vital sign data.

Based on this implementation, the vital sign data may include the first vital sign data and the second vital sign data, and the time periods for collecting the first vital sign data and the second vital sign data are different. Therefore, the first vital sign data and the second vital sign data may be used to improve diversity of the collected vital sign data, and reflect postprandial blood glucose of the user in different time periods, improving accuracy of the blood glucose assessment. For example, if the first time period corresponding to the first vital sign data is a time period in daytime, and the second time period corresponding to the second vital sign data is a time period at night, postprandial blood glucose of the user after breakfast, lunch, and afternoon tea may be reflected based on the first vital sign data, postprandial blood glucose of the user after supper may be reflected based on the second vital sign data.

In addition, according to the foregoing method, blood glucose assessment may be performed on the user only when the first cumulative total duration is greater than the threshold and the second cumulative total duration is greater than the second threshold. In this way, there may be sufficient first valid vital sign data and second valid vital sign data during the blood glucose assessment, improving the accuracy of the blood glucose assessment.

With reference to the first aspect, in a possible implementation, duration of the first cycle is one in a preset duration range.

Based on this implementation, because the preset duration range includes a minimum value and a maximum value, when the duration of the first cycle is one in the preset duration range, the duration of the first cycle is at least the minimum value, and this can avoid, based on the minimum value, a case in which an amount of the valid vital sign data is extremely small due to excessively short duration of the first cycle; and the duration of the first cycle is at most the maximum value, and this can avoid, based on the maximum value, a case in which valid vital sign data collected in a previous stage of the cycle is invalid due to excessively long duration of the first cycle.

With reference to the first aspect, in a possible implementation, the method further includes: receiving a first operation of the user in the first cycle; and in response to the first operation, displaying blood glucose assessment progress corresponding to the first cycle and/or a daily wear status of the wearable device, where the daily wear status indicates whether daily effective wear duration for the wearable device in the first cycle meets a standard.

Based on this implementation, in the blood glucose assessment process, the user may actively initiate an operation to view blood glucose assessment progress and/or a daily wear status, learn of assessment progress of a current cycle in a timely manner by viewing the blood glucose assessment progress, and learn whether the daily effective wear duration of the user meets the standard by viewing the daily wear status.

With reference to the first aspect, in a possible implementation, before the displaying blood glucose assessment progress corresponding to the first cycle and/or a daily wear status of the wearable device, the method further includes: determining a wear status of a previous day, where the wear status of the previous day indicates whether effective wear duration for the wearable device on one day before receiving of the first operation meets the standard; and if the wear status of the previous day indicates that the effective wear duration for the wearable device on one day before receiving of the first operation does not meet the standard, displaying the wear status of the previous day.

Based on this implementation, when the wear status of the previous day indicates that the effective wear duration does not meet the standard, the user may be reminded in a timely manner, so that the user can adjust a wear habit as soon as possible.

With reference to the first aspect, in a possible implementation, after the receiving a first operation of the user in the first cycle, and before the displaying blood glucose assessment progress corresponding to the first cycle and/or a daily wear status of the wearable device, the method further includes: determining whether a blood glucose assessment result of a previous cycle has been displayed to the user; and if the blood glucose assessment result of the previous cycle has not been displayed to the user, displaying the blood glucose assessment result of the previous cycle.

Based on this implementation, when the user does not view the blood glucose assessment result of the previous cycle, the blood glucose assessment result of the previous cycle may be displayed to the user in a timely manner.

With reference to the first aspect, in a possible implementation, the method further includes: when the blood glucose assessment progress corresponding to the first cycle reaches preset progress, displaying the blood glucose assessment progress corresponding to the first cycle and to-be-worn effective duration, where the to-be-worn effective duration indicates duration for which the user still needs to effectively wear the wearable device.

Based on this implementation, in the blood glucose assessment process, the blood glucose assessment progress and the to-be-worn effective duration may be actively displayed to the user, to encourage the user to persist in wearing the wearable device.

With reference to the first aspect, in a possible implementation, after the displaying the blood glucose assessment result corresponding to the first cycle, the method further includes: obtaining a result feedback for the blood glucose assessment result corresponding to the first cycle, where the result feedback indicates that the blood glucose assessment result corresponding to the first cycle is different from an actual blood glucose assessment result; and updating a blood glucose assessment model based on the result feedback, where the blood glucose assessment model is used to determine a blood glucose assessment result based on the valid vital sign data in the vital sign data.

Based on this implementation, when the blood glucose assessment result is inaccurate (that is, the blood glucose assessment result corresponding to the first cycle is different from the actual blood glucose assessment result), the blood glucose assessment model may be updated by using the result feedback, so that updated blood glucose assessment model can obtain a more accurate blood glucose assessment result.

With reference to the first aspect, in a possible implementation, before the displaying the blood glucose assessment result corresponding to the first cycle, the method further includes: if the blood glucose assessment result corresponding to the first cycle indicates that a risk level of hyperglycemia is a high risk and/or indicates that the user is among a diabetic population, displaying hyperglycemia high-risk reminder information and/or hyperglycemia health reminder information.

Based on this possible manner, when it is determined that the risk level of hyperglycemia is a high risk or the user is among the diabetic population, the user may be notified in a timely manner, to avoid missing a diagnosis and treatment opportunity.

With reference to the first aspect, in a possible implementation, the displaying the blood glucose assessment result corresponding to the first cycle includes: displaying a view reminder pop-up window for the blood glucose assessment result; and in response to a second operation on the view reminder pop-up window, displaying the blood glucose assessment result corresponding to the first cycle.

Based on this possible manner, the view reminder pop-up window may be used to remind the user to view the blood glucose assessment result. The user may initiate a corresponding operation on the view reminder pop-up window based on a current status (for example, the user is currently idle), to display the blood glucose assessment result corresponding to the first cycle.

According to a second aspect, an embodiment of this application provides an electronic device, including one or more processors and one or more memories. The one or more memories are coupled to the one or more processors. The one or more memories are configured to store computer program code, and the computer program code includes computer instructions. When the one or more processors execute the computer instructions, the electronic device is enabled to perform the method according to any one of the possible implementations of the first aspect.

According to a third aspect, an embodiment of this application provides a computer-readable storage medium, where the computer-readable storage medium stores a computer program, the computer program includes program instructions, and when the program instructions are run on an electronic device, the electronic device is enabled to perform the method according to any one of the possible implementations of the first aspect.

According to a fourth aspect, an embodiment of this application provides a computer program. The computer program includes instructions. When the computer program is executed by a computer, the computer is enabled to perform the method according to any one of the possible implementations of the first aspect.

According to a fifth aspect, an embodiment of this application provides a computer program product. When the computer program product is run on a computer, the computer is enabled to perform the method according to any one of the possible implementations of any one of the foregoing aspects.

### BRIEF DESCRIPTION OF DRAWINGS

FIG. 1 is a diagram of an architecture of a communication system according to an embodiment of this application;
FIG. 2 is a diagram of a hardware structure of an electronic device according to an embodiment of this application;
FIG. 3A-1 to FIG. 3A-3 to FIG. 3E are schematic flowcharts of some user authorization manners according to an embodiment of this application;

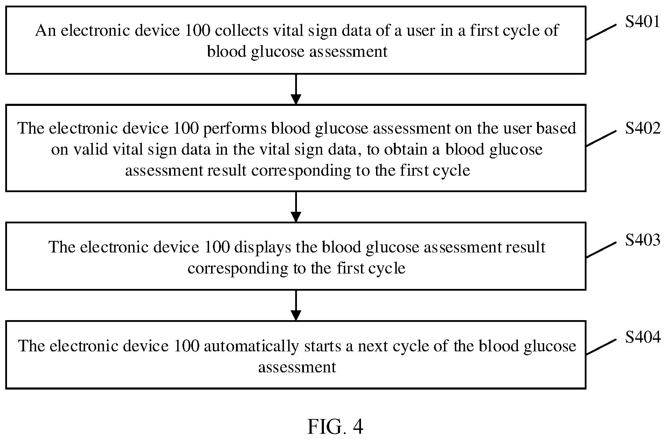

FIG. 4 is a schematic flowchart of a blood glucose assessment method according to an embodiment of this application;

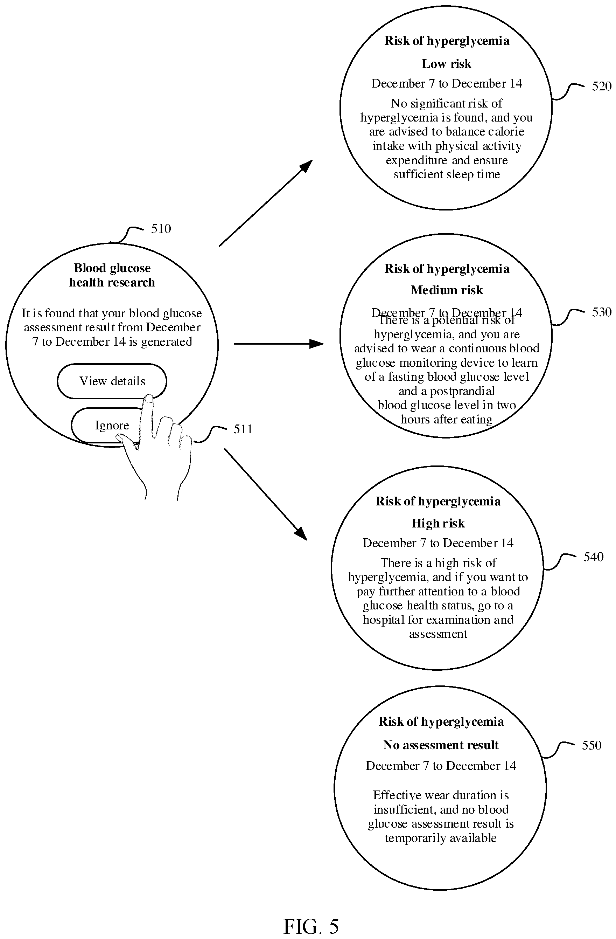

FIG. 5 is a diagram of some blood glucose assessment result interfaces according to an embodiment of this application;

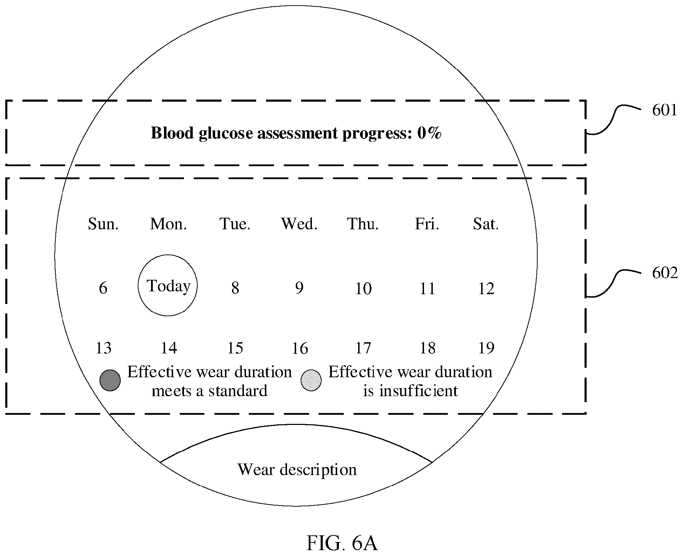

FIG. 6A to FIG. 6C are diagrams of some interfaces of blood glucose assessment progress and daily wear statuses according to an embodiment of this application;

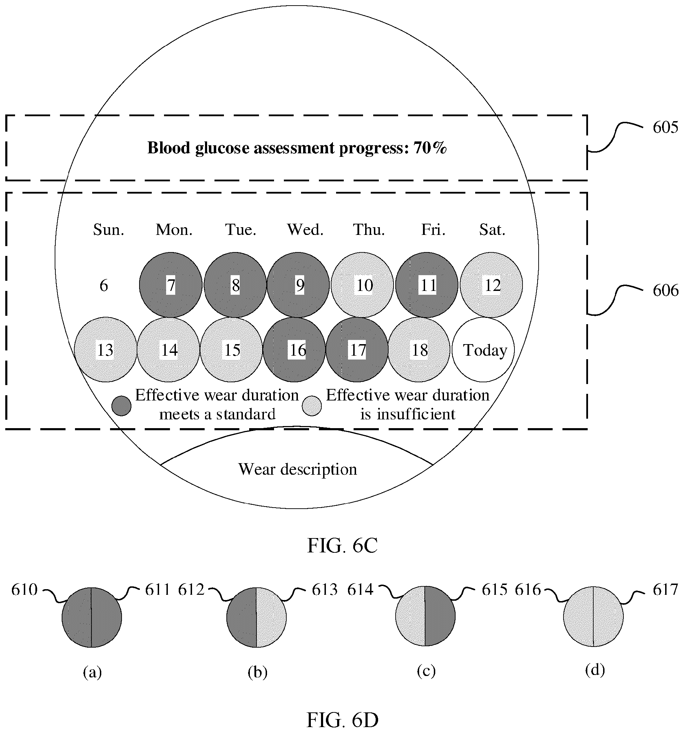

FIG. 6D is a diagram of daily wear status interfaces according to an embodiment of this application;

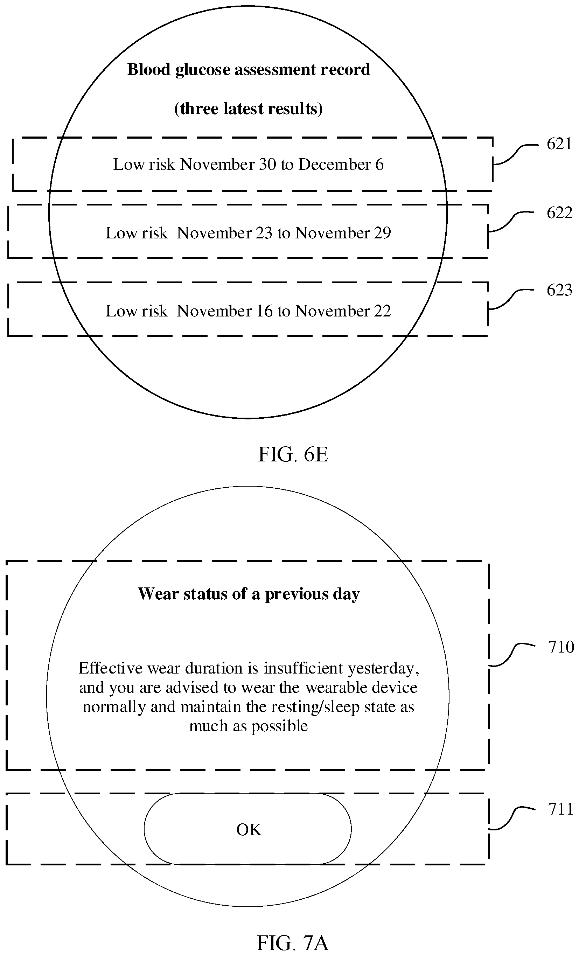

FIG. 6E is a diagram of a blood glucose assessment record interface according to an embodiment of this application;
FIG. 7A is a diagram of a reminder interface of a wear status of a previous day according to an embodiment of this application;

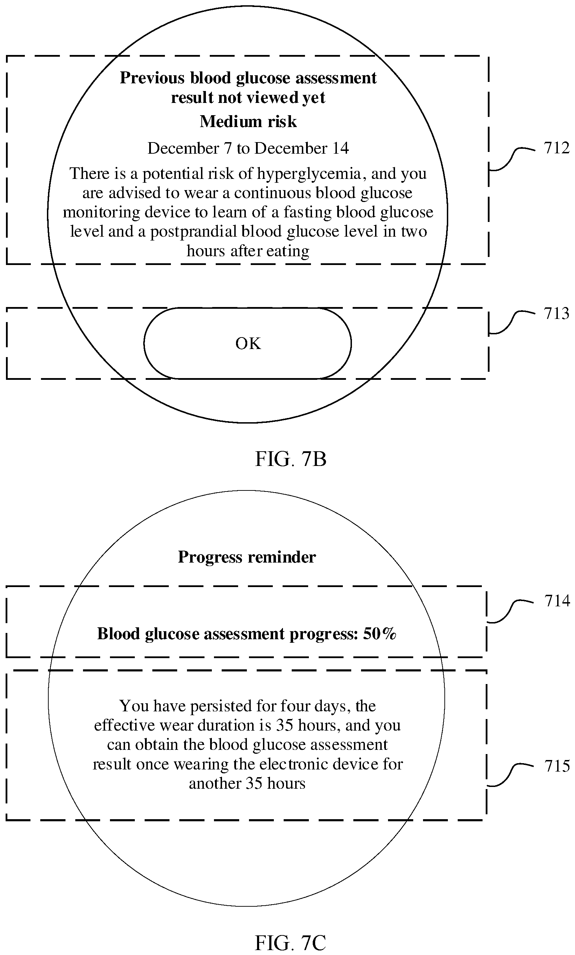

FIG. 7B is a diagram of a reminder interface for viewing a blood glucose assessment result according to an embodiment of this application;

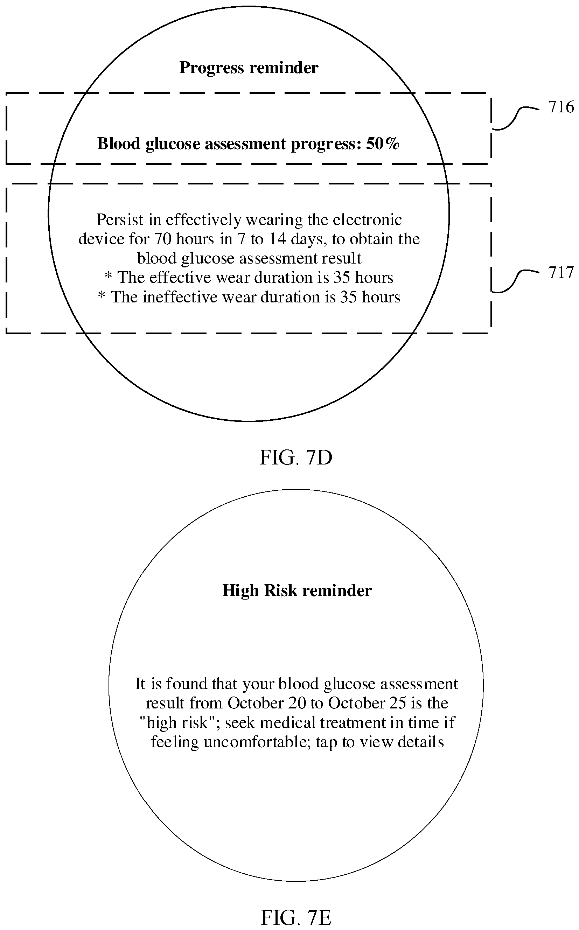

FIG. 7C and FIG. 7D are diagrams of reminder interfaces of blood glucose assessment progress according to an embodiment of this application;
FIG. 7E is a diagram of a reminder interface of a high risk according to an embodiment of this application;

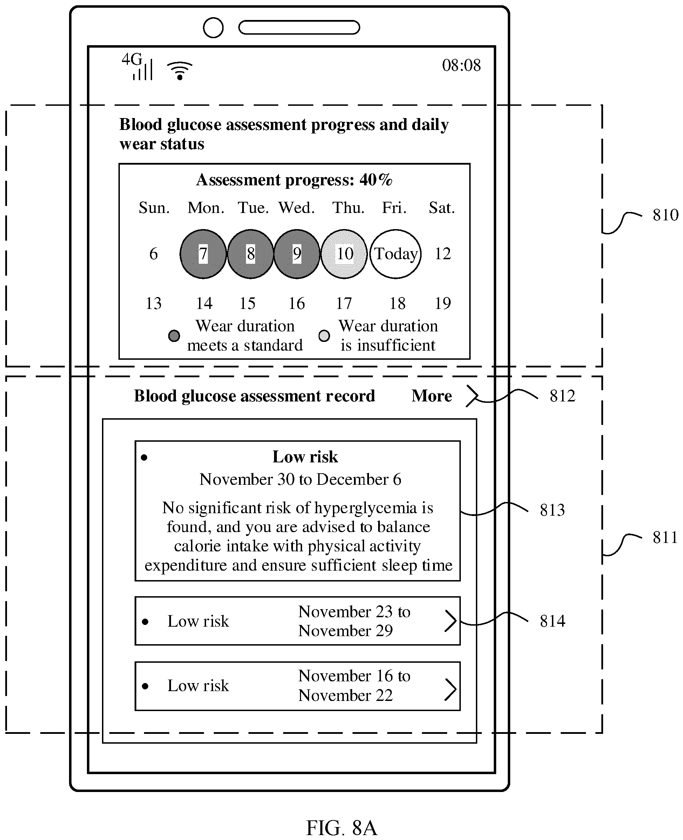

FIG. 8A is a diagram of some interfaces of blood glucose assessment progress and daily wear statuses, and a blood glucose assessment record according to an embodiment of this application;

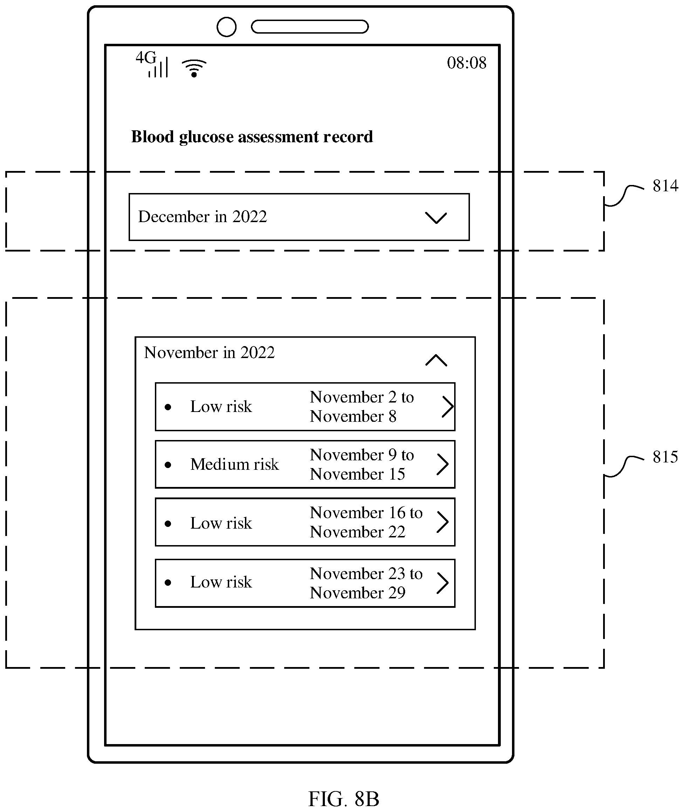

FIG. 8B is a diagram of another blood glucose assessment record interface according to an embodiment of this application;

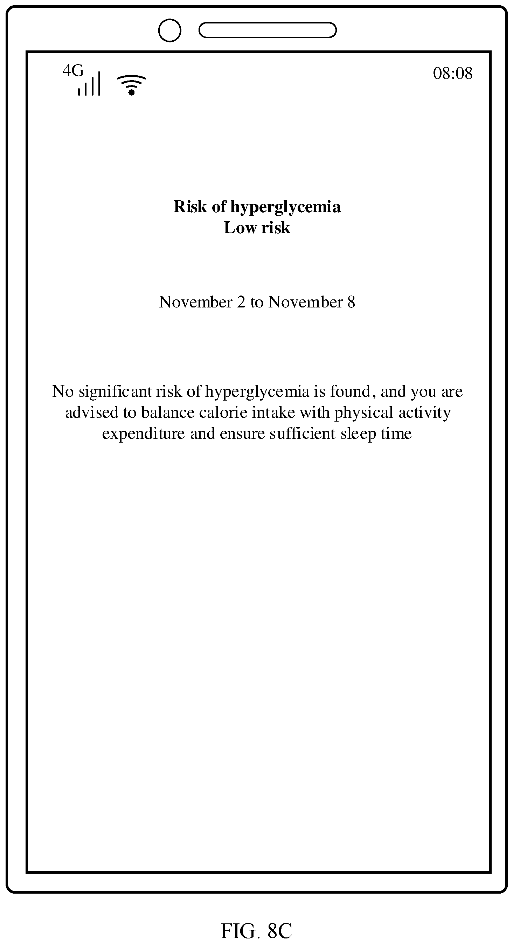

FIG. 8C is a diagram of another blood glucose assessment result interface according to an embodiment of this application;

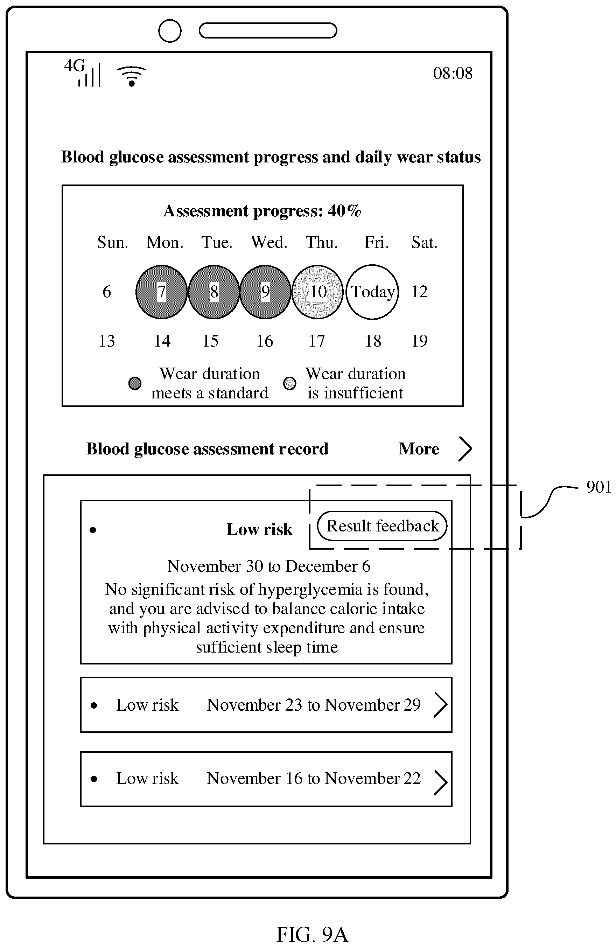

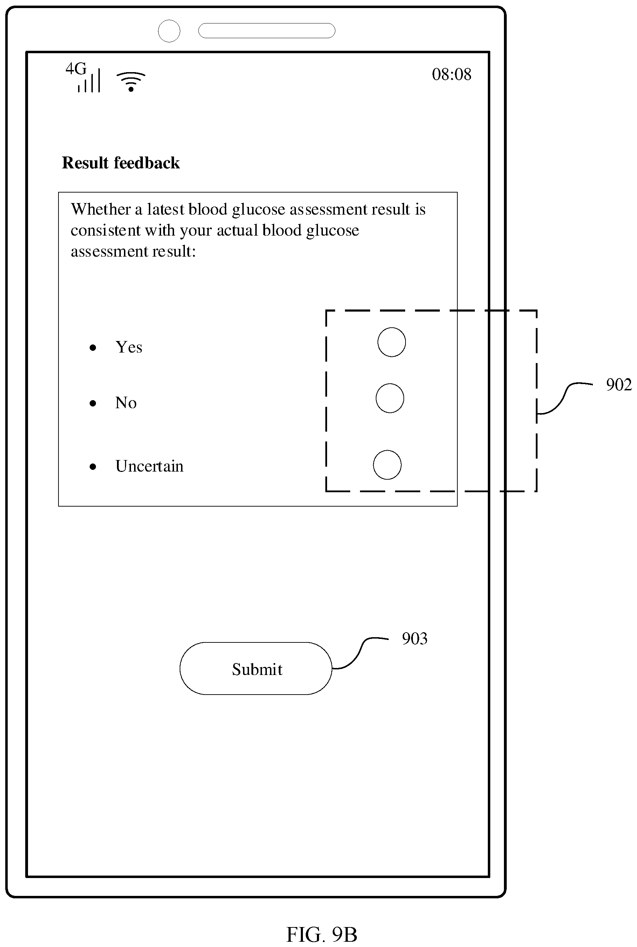

FIG. 9A and FIG. 9B are a diagram of some result feedback interfaces according to an embodiment of this application; and

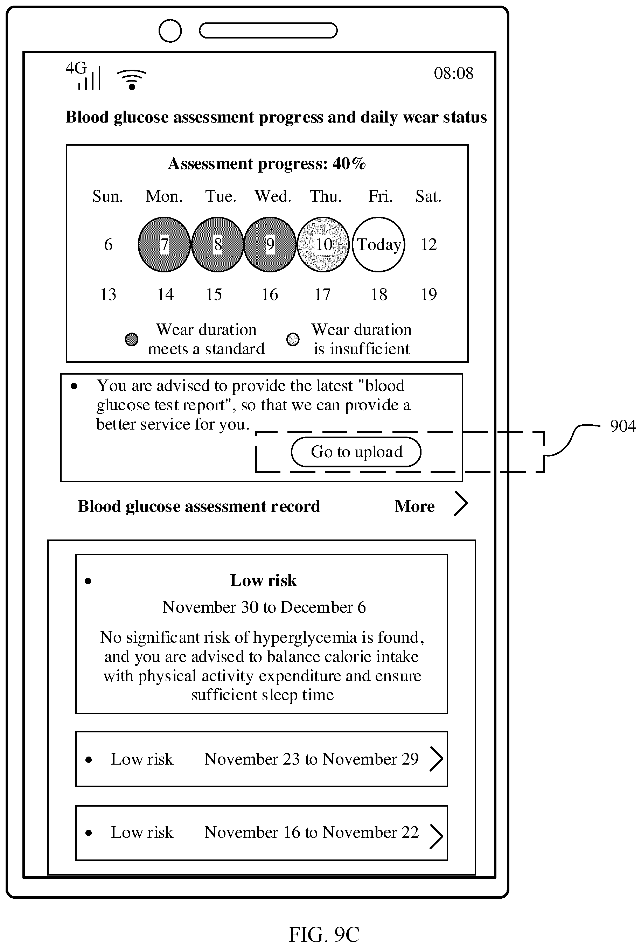

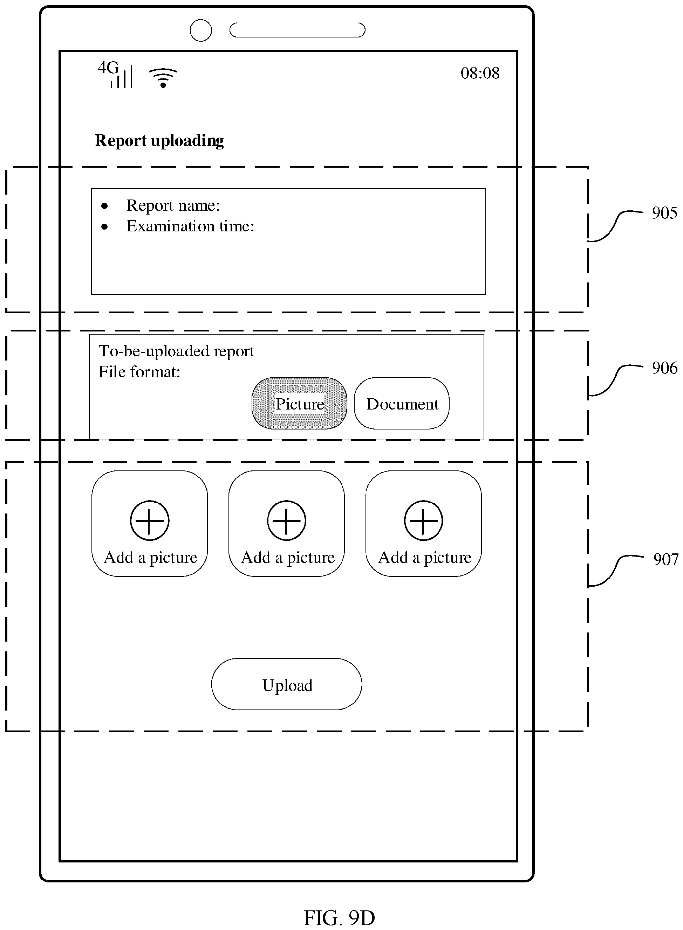

FIG. 9C and FIG. 9D are a diagram of some report uploading interfaces according to an embodiment of this application.

### DESCRIPTION OF EMBODIMENTS

The following describes embodiments of this application with reference to the accompanying drawings in embodiments of this application.

In the specification, claims, and accompanying drawings of this application, the terms "first", "second", "third", "fourth" and the like are intended to distinguish between different objects but do not indicate a particular order. In addition, the terms "including" and "having" and any other variants thereof are intended to cover a non-exclusive inclusion. For example, a process, a method, a system, a product, or a device that includes a series of steps or units is not limited to the listed steps or units, but optionally further includes an unlisted step or unit, or optionally further includes another inherent step or unit of the process, the method, the product, or the device.

It should be understood that in this application, "at least one (item)" means one or more and "a plurality of" means two or more. The term "and/or" is used for describing an association relationship between associated objects, and indicates that three relationships may exist. For example, "A and/or B" may indicate the following three cases: Only A exists, only B exists, and both A and B exist, where A and B may be singular or plural. The character "/" generally indicates an "or" relationship between the associated objects. The expression "at least one of the following items (pieces)" or a similar expression means any combination of these items, including a single item (piece) or any combination of a plurality of items (pieces). For example, at least one item (piece) of a, b, or c may indicate a, b, c, a and b, a and c, b and c, or a, b, and c, where a, b, and c may be singular or plural.

For ease of understanding embodiments of this application, terms in embodiments of this application are first described below.

### Photoplethysmography (photoplethysmography, PPG) technology

The PPG technology is an application of an infrared nondestructive detection technology in biomedicine. In the PPG technology, a PPG sensor is used to detect different intensity of reflected light that is absorbed by human blood and tissues, a change of a blood vessel volume in a cardiac cycle is described based on the different intensity of the reflected light, to obtain a pulse waveform, and physiological parameters such as a heart rate and blood oxygen are obtained based on the pulse waveform. Because these physiological parameters may reflect a physiological change related to diabetes, this application provides a blood glucose assessment method based on the PPG sensor, to periodically and automatically assess blood glucose of a user.

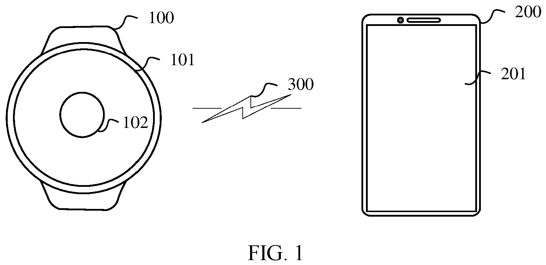

The blood glucose assessment method provided in an embodiment of this application may be applied to a communication system shown in FIG. 1. As shown in FIG. 1, the communication system includes an electronic device 100, an electronic device 200, and a communication network 300. The electronic device 100 and the electronic device 200 may communicate based on the communication network 300.

The electronic device 100 includes a display 101 and a PPG sensor 102. The display 101 is configured to receive an operation of a user and display a related interface of blood glucose assessment. For example, in this application, the blood glucose assessment related interface includes but is not limited to: one or more of a blood glucose assessment result display interface, a blood glucose assessment progress display interface, a wear status display interface, a user authorization interface, a blood glucose assessment result feedback interface, and the like. Any two or more interfaces in the foregoing example may be a same interface. This is not limited in this application. The operation of the user may be an operation used to trigger display of these related interfaces, or an operation on these related interfaces.

The PPG sensor 102 is configured to collect vital sign data of the user, where the vital sign data includes a pulse waveform (also referred to as a PPG waveform). For example, the PPG sensor 102 may be disposed on a back side of the electronic device 100. The back side means a side on which the electronic device 100 is attached to skin of the user.

The electronic device 200 includes a display 201. The display 201 is configured to receive the operation of the user and display the related interface of the blood glucose assessment. Optionally, the electronic device 100 may send related content of the blood glucose assessment to the electronic device 200 through the communication network 300, so that the electronic device 200 displays the related interface of the blood glucose assessment. For example, the electronic device 100 may send one or more of a blood glucose assessment result, a wear status, blood glucose assessment progress, a result feedback, and the like to the electronic device 200 based on Bluetooth communication, so that the electronic device 200 displays one or more of the blood glucose assessment result display interface, the blood glucose assessment progress display interface, the wear status display interface, the blood glucose assessment result feedback interface, and the like.

In other words, in a blood glucose assessment process, the user may view the related content of the blood glucose assessment by using the electronic device 100 or the electronic device 200, and the related content may be displayed by using the related interface of the blood glucose assessment.

For example, the electronic device 100 and the electronic device 200 each may be a portable terminal device carrying iOS^{®}, Android^{®}, Microsoft^{®}, harmony^{®}, or another operating system. The electronic device 100 and the electronic device 200 each may be a mobile phone, a tablet computer, a desktop computer, a laptop computer, a handheld computer, a notebook computer, an ultra-mobile personal computer (ultra-mobile personal computer, UMPC), a netbook, a cellular phone, a personal digital assistant (personal digital assistant, PDA), an augmented reality (augmented reality, AR) device, a virtual reality (virtual reality, VR) device, an artificial intelligence (artificial intelligence, AI) device, a wearable device, a vehicle, a vehicle-mounted device, a smart home device, and/or a smart city device, which is not limited thereto, and may further include a non-portable terminal device like a laptop computer (laptop) having a touch-sensitive surface or a touch panel, a desktop computer having a touch-sensitive surface or a touch panel, or the like. Specific types of the electronic device 100 and the electronic device 200 are not specially limited in implementations of this application.

For example, the electronic device 100 may be a wearable device. The wearable device may include but is not limited to: a watch or a band worn on a wrist of the user, a close-fitting watch, a ring worn on a finger of the user, an in-ear earphone, a headphone, smart close-fitting clothing, a smart close-fitting accessory, and the like. The electronic device 200 may be a smartphone.

For example, the communication network 300 may be a local area network (local area networks, LAN), or may be a wide area network (wide area networks, WAN), for example, the internet. The communication network 300 may be implemented by using any known network communication protocol. The network communication protocol may be various wired or wireless communication protocols, such as Ethernet, universal serial bus (universal serial bus, USB), a firewire (FIREWIRE), long term evolution (long term evolution, LTE), Bluetooth, wireless fidelity (wireless fidelity, Wi-Fi), near field communication (near field communication, NFC), a voice over internet protocol (voice over Internet protocol, VoIP), a communication protocol that supports a network slicing architecture, or any other suitable communication protocol.

It should be noted that the blood glucose assessment method provided in this embodiment of this application may also be independently applied to the electronic device 100.

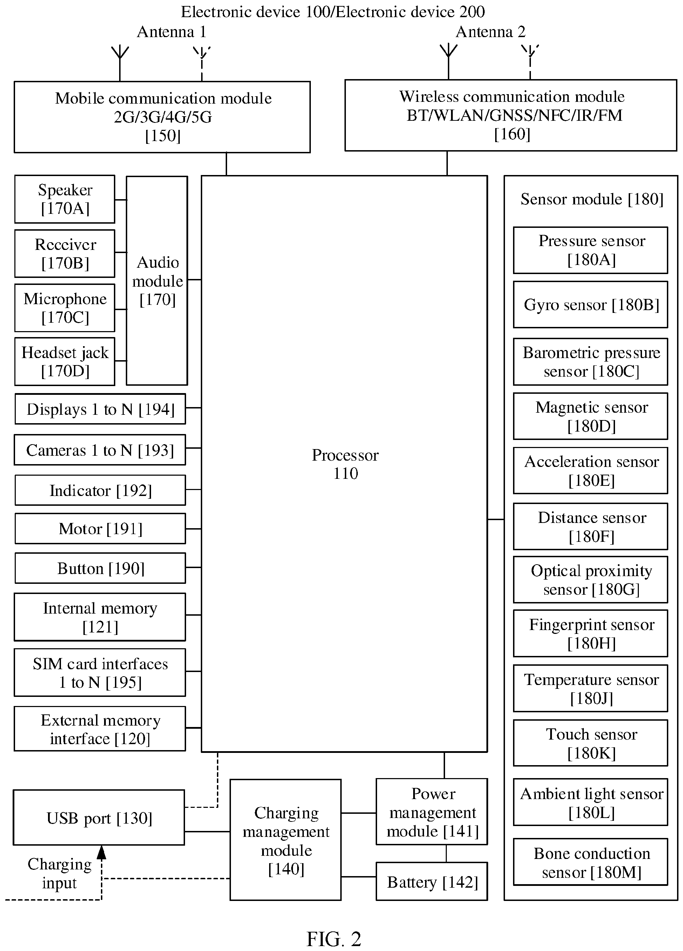

FIG. 2 is a diagram of a hardware structure of an electronic device 100/electronic device 200 according to an embodiment of this application. For example, the electronic device 100/electronic device 200 may include a processor 110, an external memory interface 120, an internal memory 121, a universal serial bus (universal serial bus, USB) port 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like. The sensor module 180 may include a pressure sensor 180A, a gyro sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of the present invention does not constitute a specific limitation on the electronic device 100/electronic device 200. In some other embodiments of this application, the electronic device 100/electronic device 200 may include more or fewer components than those shown in the figure, or some components may be combined, or some components may be split, or different component arrangements may be used. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent devices, or may be integrated into one or more processors.

The controller may generate an operation control signal based on an instruction operation code and a time sequence signal, to complete control of instruction fetch and instruction execution.

A memory may be further disposed in the processor 110, and is configured to store instructions and data. In some embodiments, the memory in the processor 110 is a cache memory. The memory may store an instruction or data that has been used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) port, and/or the like.

The charging management module 140 is configured to receive a charging input from a charger. The charger may be a wireless charger or a wired charger. In some embodiments of wired charging, the charging management module 140 may receive a charging input of a wired charger through the USB port 130. In some embodiments of wireless charging, the charging management module 140 may receive a wireless charging input through a wireless charging coil of the electronic device 100/electronic device 200. The charging management module 140 supplies power to the electronic device through the power management module 141 while charging the battery 142.

The power management module 141 is configured to connect to the battery 142, the charging management module 140, and the processor 110. The power management module 141 receives an input from the battery 142 and/or the charging management module 140, and supplies power to the processor 110, the internal memory 121, the display 194, the camera 193, the wireless communication module 160, and the like. The power management module 141 may be further configured to monitor a parameter like a battery capacity, a battery cycle count, or a battery health status (electric leakage or impedance). In some other embodiments, the power management module 141 may alternatively be disposed in the processor 110. In some other embodiments, the power management module 141 and the charging management module 140 may alternatively be disposed in a same device.

A wireless communication function of the electronic device 100/electronic device 200 may be implemented by using the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna on the electronic device 100/electronic device 200 may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a wireless communication solution that is applied to the electronic device 100/electronic device 200 and that includes 2G, 3G, 4G, 5G, and the like. The mobile communication module 150 may include at least one filter, a switch, a power amplifier, a low noise amplifier (low noise amplifier, LNA), and the like. The mobile communication module 150 may receive an electromagnetic wave by using the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation by using the antenna 1. In some embodiments, at least some functional modules in the mobile communication module 150 may be disposed in the processor 110. In some embodiments, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (not limited to the speaker 170A, the receiver 170B, and the like), and displays an image or a video through the display 194. In some embodiments, the modem processor may be an independent device. In some other embodiments, the modem processor may be independent of the processor 110, and is disposed in a same device as the mobile communication module 150 or another functional module.

The wireless communication module 160 may provide a wireless communication solution that is applied to the electronic device 100/electronic device 200 and that includes a wireless local area network (wireless local area networks, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more devices integrating at least one communication processing module. The wireless communication module 160 receives an electromagnetic wave by using the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation by using the antenna 2.

In some embodiments, the antenna 1 and the mobile communication module 150 on the electronic device 100/electronic device 200 are coupled, and the antenna 2 and the wireless communication module 160 on the electronic device 100/electronic device 200 are coupled, so that the electronic device 100/electronic device 200 can communicate with a network and another device by using a wireless communication technology. The wireless communication technology may include a global system for mobile communications (global system for mobile communications, GSM), a general packet radio service (general packet radio service, GPRS), long term evolution (long term evolution, LTE), BT, GNSS, WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation systems, SBAS).

The electronic device 100/electronic device 200 implements a display function by using the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs, which execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flexible light-emitting diode, FLED), a Miniled, a MicrOLED, a Micro-OLED, a quantum dot light emitting diode (quantum dot light emitting diodes, QLED), or the like. In some embodiments, the electronic device 100/electronic device 200 may include one or N displays 194, where N is a positive integer greater than 1.

The electronic device 100/electronic device 200 may implement an image shooting function by using the ISP, the camera 193, the video codec, the GPU, the display 194, the application processor, and the like.

The digital signal processor is configured to process a digital signal, and may process another digital signal in addition to the digital image signal. For example, when the electronic device 100/electronic device 200 selects a frequency point, the digital signal processor is configured to perform Fourier transformation or the like on frequency point energy.

The video codec is configured to compress or decompress a digital video. The electronic device 100/electronic device 200 may support one or more video codecs. Therefore, the electronic device 100/electronic device 200 may play or record videos in a plurality of coding formats, for example, moving picture experts group (moving picture experts group, MPEG)1, MPEG2, MPEG3, and MPEG4.

The NPU is a neural-network (neural-network, NN) computing processor. The NPU quickly processes input information with reference to a structure of a biological neural network, for example, a transfer mode between human brain neurons, and may further continuously perform self-learning. Applications such as intelligent cognition of the electronic device 100/electronic device 200 may be implemented through the NPU, for example, image recognition, facial recognition, speech recognition, and text understanding.

The internal memory 121 may be one or more random access memories (random access memory, RAM), and one or more non-volatile memories (non-volatile memory, NVM).

The random access memory may include a static random-access memory (static random-access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, a DDR SDRAM, like a 5th generation DDR SDRAM, usually referred to as a DDR5 SDRAM), or the like. The non-volatile memory may include a magnetic disk storage device and a flash memory (flash memory).

The flash memory may be classified into an NOR flash, an NAND flash, a 3D NAND flash, and the like according to an operation principle; may be classified into a single-level cell (single-level cell, SLC), a multi-level cell (multi-level cell, MLC), a triple-level cell (triple-level cell, TLC), a quad-level cell (quad-level cell, QLC), and the like based on a quantity of electric potential levels of a cell; or may be classified into a universal flash storage (English: universal flash storage, UFS), an embedded multi media card (embedded multi media card, eMMC), and the like according to storage specifications.

The random access memory may be directly read and written by the processor 110, may be configured to store executable programs (such as machine instructions) of an operating system or another running program, and may also be configured to store data of a user and applications.

The non-volatile memory may also store the executable programs, the data of the user and the applications, and the like, and may be loaded into the random access memory in advance, to be directly read and written by the processor 110.

The external memory interface 120 may be configured to connect to an external non-volatile memory, to expand a storage capability of the electronic device 100/electronic device 200. The external non-volatile memory communicates with the processor 110 through the external memory interface 120, to implement a data storage function. For example, files such as music and videos are stored in the external non-volatile memory.

The electronic device 100/electronic device 200 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert analog audio input into a digital audio signal. The audio module 170 may be further configured to code and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an electrical audio signal into a sound signal.

The receiver 170B, also referred to as an "earpiece", is configured to convert an electrical audio signal into a sound signal.

The microphone 170C, also referred to as a "mike" or a "mic", is configured to convert a sound signal into an electrical signal.

The headset jack 170D is configured to connect to a wired headset.

The button 190 includes a power button, a volume button, and the like. The button 190 may be a mechanical button, or may be a touch button. The electronic device 100/electronic device 200 may receive a button input, and generate a button signal input related to user setting and function control of the electronic device 100/electronic device 200.

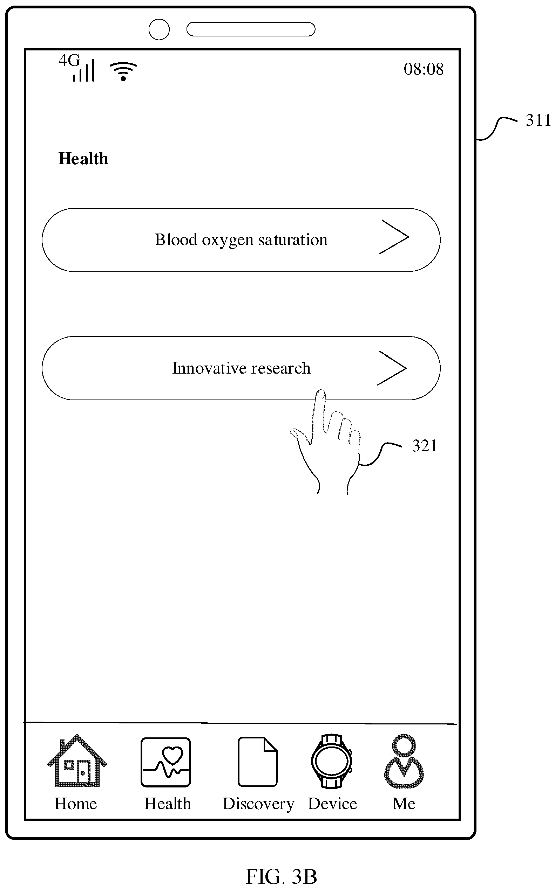

Optionally, before blood glucose assessment is performed on the user, user authorization needs to be obtained first, and the automatic blood glucose assessment is periodically and automatically started after the user authorization is obtained. The following uses FIG. 3A-1 to FIG. 3A-3 to FIG. 3E as examples to first describe user authorization manners during blood glucose assessment. A procedure shown in FIG. 3A-1 to FIG. 3A-3 may be implemented based on an electronic device 100, and a procedure shown in FIG. 3B to FIG. 3E may be implemented based on an electronic device 200.

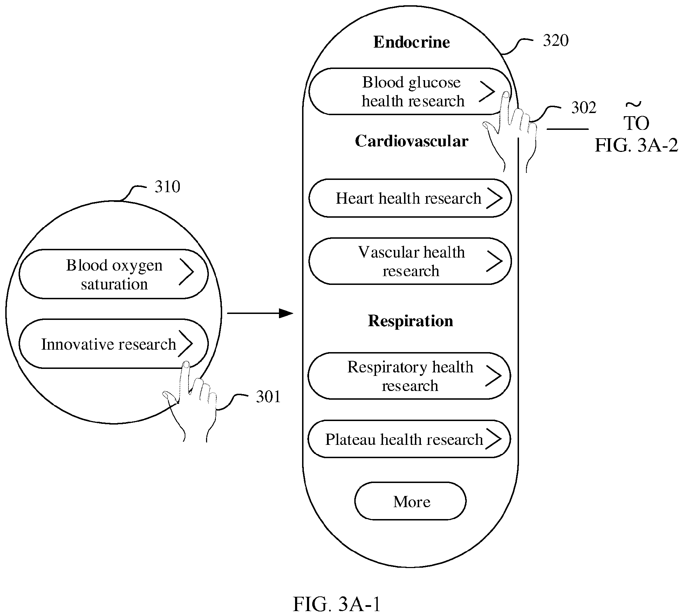

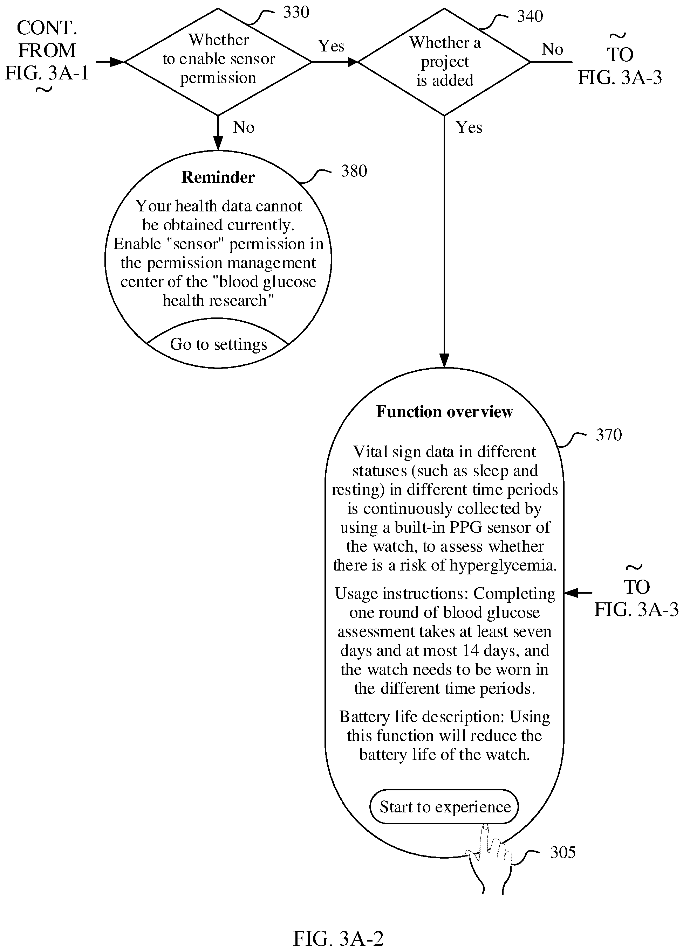

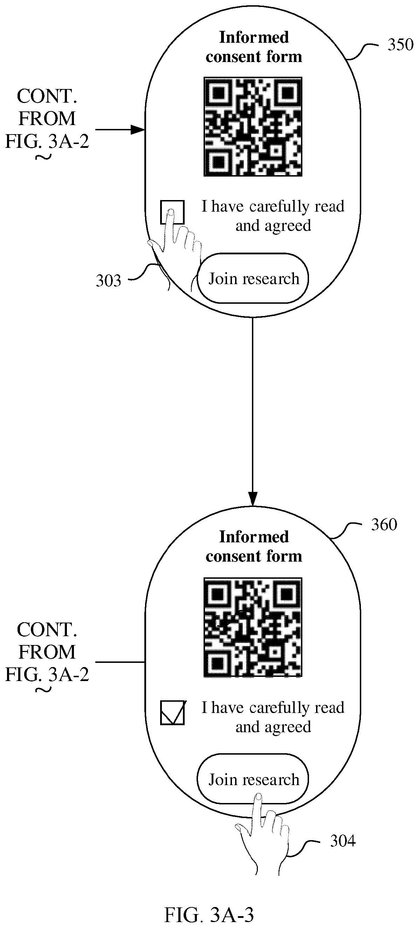

FIG. 3A-1 to FIG. 3A-3 is a schematic flowchart of a user authorization manner according to an embodiment of this application. The electronic device 100 may display an interface 310, and the interface 310 includes blood oxygen saturation and innovative research. When receiving an operation 301 on the innovative research, the electronic device 100 may display an interface 320. The interface 320 includes endocrine research, cardiovascular research, respiratory research, and more types of research. It should be noted that, because the electronic device 100 is limited by a size of a display, the interface 320 may alternatively include only the cardiovascular research. A user may view the endocrine research by performing a slide-up operation, and view the respiratory research and more types of research by performing a slide-down operation. The interface 320 in FIG. 3A-1 shows all types of research that can be displayed by the electronic device 100 through a slide operation of the user. Among the research, the endocrine research includes blood glucose health research, the cardiovascular research includes heart health research and vascular health research, and the respiratory research includes respiratory health research and plateau health research. When the electronic device 100 receives an operation 302 on the blood glucose health research, the electronic device 100 determines whether sensor permission is enabled (that is, determines whether a PPG sensor is started). When determining that the sensor permission is not enabled, the electronic device 100 may display an interface 380. The interface 380 includes reminder information (namely, your health data cannot be obtained currently, please enable the "sensor" permission in the "blood glucose health research" permission management center) and a "go to settings" control. The user may operate the "go to settings" control to enable the sensor permission. When the electronic device 100 determines that the sensor permission is enabled, the electronic device 100 determines whether a project is added. Whether a project is added means whether the user decides to participate in the blood glucose health research.

When the electronic device 100 determines that the electronic device 100 the project is not added, the electronic device 100 displays an interface 350. The interface 350 includes an informed consent form, an "agree" control in the informed consent form, and a "join research" control. For example, the informed consent form may be displayed in a form of a two-dimensional code, a text, or a picture. The interface 350 shows the display form of the two-dimensional code. The user may scan the two-dimensional code by using a device like an intelligent terminal, to view specific content of the informed consent form. When the user finishes reading the informed consent form, the user may initiate an operation 303 on the "agree" control. After receiving the operation 303, the electronic device 100 displays an interface 360. In the interface 360, when the electronic device 100 receives an operation 304 on the "join research" control, the electronic device 100 may display an interface 370. The interface 370 includes a function overview of the blood glucose health research and a "start to experience" control. The function overview includes a function introduction (namely, vital sign data in different statuses (such as sleep and resting) in different time periods is continuously collected by using a built-in PPG sensor of the watch, to assess whether there is a risk of hyperglycemia), usage instructions (namely, completing one round of blood glucose assessment takes at least 7 days and at most 14 days, and the watch needs to be worn in the different time periods), and a battery life description (namely, using this function will reduce the battery life of the watch). When the electronic device 100 receives an operation 305 on the "start to experience" control in the interface 370, the electronic device 100 may start the blood glucose assessment after obtaining user authorization.

When the electronic device 100 determines that the project is added, the electronic device 100 directly displays the interface 370. When the electronic device 100 receives an operation 305 on the "start to experience" control in the interface 370, the electronic device 100 may start the blood glucose assessment after obtaining user authorization.

It may be understood that the interface 310 to the interface 370 are merely examples, and more or less content may be further included during specific implementation. This is not limited in this application. In addition, each of the operation 301 to the operation 305 may be a touch control operation in any form, for example, tap, or touch and hold. This is not limited in this application. Alternatively, if the electronic device 100 enables a speech recognition function, or the user sets, on the electronic device 100, a shortcut operation for obtaining the user authorization, or the like, the user may enable, by using the speech recognition function or the shortcut operation, the electronic device 100 to obtain the user authorization and start the blood glucose assessment.

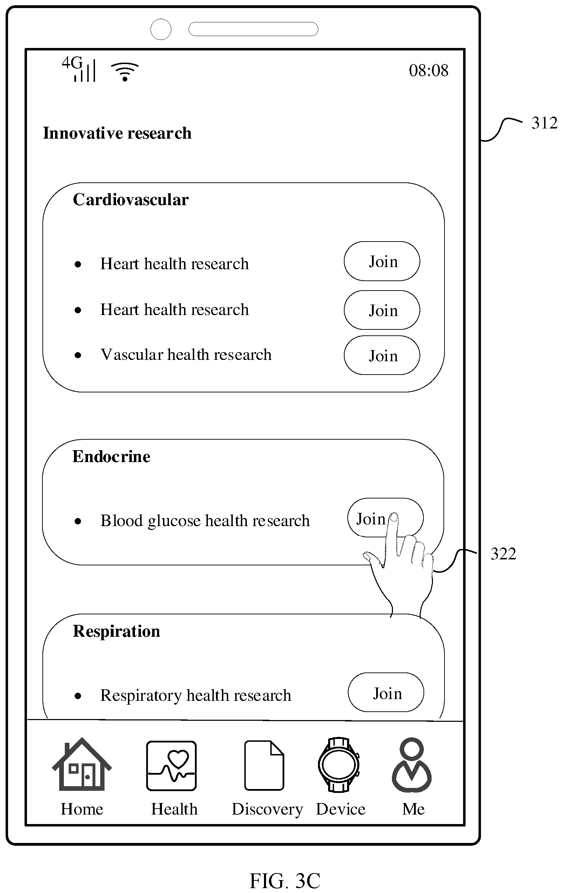

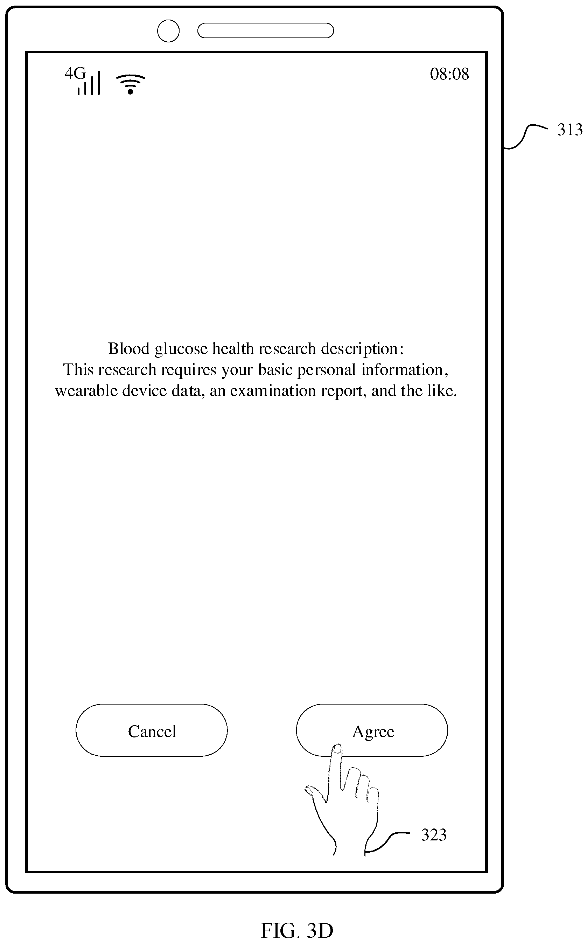

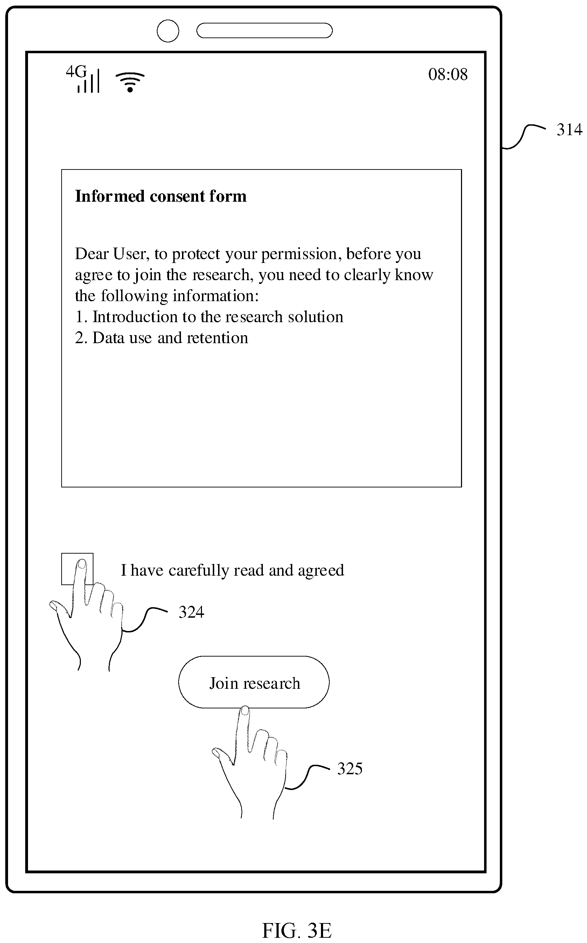

FIG. 3B to FIG. 3E are schematic flowcharts of another user authorization manner according to an embodiment of this application. An electronic device 200 may display an interface 311 shown in FIG. 3B. The interface 311 shows an example of a main interface that is used to manage a health application of a wearable device. The interface 311 includes a status bar and a health interface. The status bar includes home, health, discovery, device, and me. The health interface includes blood oxygen saturation and innovative research. For example, the electronic device 200 may display the interface shown in FIG. 3B after a user taps health in the status bar at home of the application. When receiving an operation 321 on innovative research, the electronic device 200 may display an interface 312 shown in FIG. 3C. The interface 312 includes endocrine research, cardiovascular research, and respiratory research. It should be noted that, because an electronic device 100 is limited by a size of a display, types of research displayed in the interface 312 may be incomplete, and the user may perform a slide operation to enable the electronic device 200 to display all types of research, for example, the interface 320 shown in FIG. 3A-1. Among the research, the endocrine research includes blood glucose health research and a "join" control used to join blood glucose health research, the cardiovascular research includes heart health research, vascular health research, and corresponding "join" controls, and the respiratory research includes respiratory health research and a corresponding "join" control. When the electronic device 200 receives an operation 322 on the "join" control of the blood glucose health research, the electronic device 200 displays an interface 313 shown in FIG. 3D. The interface 313 includes a blood glucose health research description (namely, this research requires your basic personal information, wearable device data, an examination report, and the like), and a "cancel" control and an "agree" control of the description. When receiving an operation 323 performed by the user on the "agree" control, the electronic device 200 may display an interface 314 shown in FIG. 3E. The interface 314 includes an informed consent form, an "agree" control in the informed consent form, and a "join research" control. For example, the informed consent form may be displayed in a form of a two-dimensional code, a text, or a picture. The interface 314 shows the text display form (namely, dear User, to protect your permission, before you agree to join the research, you need to clearly know the following information: 1. introduction to the research solution; and 2. data use and retention). When the user finishes reading the informed consent form, the user may first initiate an operation 324 on the "agree" control, and then initiate an operation 325 on the "join research" control. After receiving the operation 325, the electronic device 200 obtains user authorization, and may notify the electronic device 100 to start blood glucose assessment.

Optionally, before displaying the interface 313 shown in FIG. 3D, the electronic device 200 may further determine whether to enable sensor permission on the electronic device 100, and display the interface 313 after determining that the sensor permission is enabled. Alternatively, after receiving the operation 323, the electronic device 200 may actively notify the electronic device 100 to enable the sensor permission, or the like. In addition, after displaying FIG. 3E, the electronic device 200 may further display the interface 370 (namely, the function overview) in FIG. 3A-2, so that the user wears the electronic device 100 based on a description in the function overview during the blood glucose assessment.

It may be understood that the interface 311 to the interface 314 are merely examples, and more or less content may be further included during specific implementation. This is not limited in this application. In addition, each of the operation 321 to the operation 325 may be a touch control operation in any form. This is not limited in this application. Alternatively, if the electronic device 200 enables a speech recognition function, or the user sets, on the electronic device 200, a shortcut operation for obtaining the user authorization, or the like, the user may enable, by using the speech recognition function, the shortcut operation, or the like, the electronic device 200 to obtain the user authorization, and notify the electronic device 100 to start the blood glucose assessment.

In summary, the user may complete authorization of the blood glucose assessment by using the electronic device 100 or the electronic device 200, to start the blood glucose assessment.

The following describes, by using FIG. 4, a blood glucose assessment method provided in this application. FIG. 4 is a schematic flowchart of a blood glucose assessment method according to an embodiment of this application. The method includes S401 to S404, and may be performed by an electronic device 100.

S401: The electronic device 100 collects vital sign data of a user in a first cycle of blood glucose assessment.

S402: The electronic device 100 performs blood glucose assessment on the user based on valid vital sign data in the vital sign data, to obtain a blood glucose assessment result corresponding to the first cycle.

The vital sign data includes a PPG signal collected by using a sensor, and the PPG signal may be represented as a pulse waveform (or referred to as a PPG waveform). This collection manner is an automatic collection manner that does not need to be triggered by the user.

In this embodiment of this application, first, after collecting the vital sign data of the user, the electronic device 100 may extract the valid vital sign data from the vital sign data. For example, the valid vital sign data may be vital sign data whose signal quality meets a requirement in the vital sign data.

In a possible implementation, the electronic device 100 may preprocess the vital sign data, to obtain the vital sign data whose signal quality meets the requirement in the vital sign data, and use the vital sign data whose signal quality meets the requirement as the valid vital sign data.

For example, a preprocessing manner includes but is not limited to one or more of the following: determining whether a similarity between an Nth PPG waveform (for example, a PPG waveform of a 100^{th} second to a 110^{th} second) and a previous PPG waveform (for example, a PPG waveform of a 90^{th} second to the 100^{th} second) that are included in the vital sign data is greater than a similarity threshold; or determining whether a heart rate feature or the like extracted from the vital sign data is within a normal range. Optionally, the electronic device 100 may perform preprocessing in real time when collecting vital sign data; or may preprocess collected vital sign data at an interval of a predetermined time period (for example, daily preprocess vital sign data collected on a current day).

Second, the electronic device 100 may perform blood glucose assessment on the user based on the valid vital sign data, to obtain the blood glucose assessment result corresponding to the first cycle.

In a possible implementation, the electronic device 100 extracts a first feature from the valid vital sign data; extracts a second feature from the first feature; and inputs the second feature into a blood glucose assessment model, to obtain the blood glucose assessment result corresponding to the first cycle.

For example, the first feature includes but is not limited to: a basic feature of the PPG waveform, a heart rate-related feature in the PPG waveform, and the like. The basic feature of the PPG waveform includes but is not limited to: a frequency domain feature, a waveform area feature, and the like of the PPG waveform. The second feature indicates a change of the first feature. For example, the second feature includes but is not limited to: a standard deviation of NN intervals (standard diviation of NN intervals, SDNN) of all sinus heart rates, PNN50 (a percentage of a quantity of differences between adjacent NNs that are greater than 50 milliseconds to a total quantity of all sinus heart rates), and the like.

The blood glucose assessment model is a pre-trained neural network model, and the blood glucose assessment model is used to output the blood glucose assessment result based on the input second feature. The blood glucose assessment result may indicate a risk level of hyperglycemia and/or a population type to which the user belongs, and the like. For example, risk levels of hyperglycemia may be classified into a high risk, a medium risk, and a low risk. The population type to which the user belongs may be classified into a diabetic population, a pre-diabetic population, and a healthy population.

Optionally, the risk level and the population type may be further classified in a more detailed manner. This is not limited in this application. For example, the risk level may be classified into four or more levels, and the population type may be classified into a diabetic population, a type 1 pre-diabetic population (pre-diabetic is determined due to abnormal fasting blood glucose), a type 2 pre-diabetic population (pre-diabetic is determined due to abnormal glucose tolerance), and a healthy population.

The following specifically describes the mentioned vital sign data and the valid vital sign data.

In a first possible implementation, the vital sign data includes first vital sign data collected in a first time period and second vital sign data collected in a second time period, and the first time period and the second time period are two time periods on a same day. The electronic device 100 may further determine first cumulative total duration and second cumulative total duration, where the first cumulative total duration is cumulative total duration for collecting first valid vital sign data in the first cycle, and the second cumulative total duration is cumulative total duration for collecting second valid vital sign data in the first cycle, the first valid vital sign data is valid vital sign data in the first vital sign data, and the second valid vital sign data is valid vital sign data in the second vital sign data. If the first cumulative total duration is greater than a first threshold, and the second cumulative total duration is greater than a second threshold, the electronic device 100 performs blood glucose assessment on the user based on the first valid vital sign data in the first vital sign data and the second valid vital sign data in the second vital sign data.

For example, the first time period may be a time period corresponding to daytime (for example, 8:00 am to 12:00 pm on a current day), and the second time period may be a time period corresponding to night (for example, 0:00 am to 8:00 am on the current day). In this case, the first vital sign data is vital sign data collected in the time period corresponding to the daytime, and the second vital sign data is data collected in the time period corresponding to the night. It should be noted that total duration of the first time period and the second time period may be 24 hours, or may not be 24 hours (for example, the first time period is 8:00 am to 8:00 pm on the current day, and the second time period is 0:00 am to 8:00 am on the current day. This manner can effectively reduce power consumption of the device, and prolong a battery life). This is not limited in this application.

In addition, in a possible implementation, the electronic device 100 may collect the first vital sign data in the first time period and/or collect the second vital sign data in the second time period in a continuous collection manner or an intermittent collection manner. For example, the electronic device 100 continuously collects vital sign data in the time period from 8:00 am to 12:00 pm on the current day, and uses all vital sign data collected in the time period as the first vital sign data collected on the current day. Alternatively, the electronic device 100 collects vital sign data at an interval of 15 minutes in the time period from 8:00 am to 12:00 pm on the current day, where duration of each collection is 10 minutes, and uses vital sign data collected a plurality of times in the time period as the first vital sign data collected on the current day.

For example, the first valid vital sign data is vital sign data whose signal quality meets the requirement in the first vital sign data, and the second valid vital sign data is vital sign data whose signal quality meets the requirement in the second vital sign data. In addition, the first valid vital sign data and the second valid vital sign data may indicate a vital sign change of the user in a resting or sleep state. Each of the first valid vital sign data and the second valid vital sign data includes a PPG signal, the PPG signal may be represented as a waveform, and there is a correspondence between the waveform and collection time. Therefore, in the first cycle, collection duration corresponding to a PPG waveform included in the collected first valid vital sign data may be used as the first cumulative total duration, and collection duration corresponding to a PPG waveform included in the collected second valid vital sign data may be used as the second cumulative total duration.

In this embodiment of this application, when the first cumulative total duration is greater than a first threshold, and the second cumulative total duration is greater than a second threshold, the electronic device 100 may perform blood glucose assessment on the user based on the first valid vital sign data and the second valid vital sign data. For example, the first cumulative total duration is 60 hours, the second cumulative total duration is 40 hours, the first threshold is 56 hours, and the second threshold is 35 hours. Because 60 hours are greater than 40 hours and 56 hours are greater than 35 hours, in this case, the electronic device may perform blood glucose assessment on the user based on the 60-hour first valid vital sign data and the 56-hour second valid vital sign data.

In a current blood glucose assessment technology, postprandial blood glucose in the resting or sleep state is a relatively accurate standard. However, an eating habit of the user is relatively unstable, and an eating time period is not necessarily the same each time. Therefore, in this application, the first vital sign data in the first time period (for example, the time period corresponding to the daytime) may be used to reflect postprandial blood glucose of the user after breakfast, lunch, afternoon tea, and the like, and the second vital sign data in the second time period (for example, the time period corresponding to the night) may be used to reflect the postprandial blood glucose of the user after supper. It can be learned that diversity of the vital sign data can be improved by collecting the vital sign data in the two time periods, helping improve accuracy of the blood glucose assessment.

In a second possible implementation, the vital sign data includes only vital sign data of the user collected in one time period. The electronic device 100 may further determine third cumulative total duration for collecting valid vital sign data in the vital sign data in the first cycle. If the third cumulative total duration is greater than a third threshold, the electronic device 100 performs blood glucose assessment on the user based on the valid vital sign data in the vital sign data.

For example, the time period may be a time period corresponding to one entire day (for example, 0:00 am to 12:00 pm on a current day), a time period corresponding to daytime (for example, 8:00 am to 12:00 pm on the current day), or a time period corresponding to night (for example, 0:00 am to 8:00 am on the current day). In addition, in a possible implementation, the electronic device 100 may collect the vital sign data in one time period in a continuous collection manner or an intermittent collection manner.

The following describes duration of the first cycle. The duration of the first cycle has the following several possible cases:
Case 1: The duration of the first cycle is preset fixed duration.

Optionally, the electronic device 100 may determine, based on whether the duration of the first cycle reaches the fixed duration, whether to perform blood glucose assessment on the user.

Optionally, the electronic device 100 may further determine, based on whether the duration of the first cycle reaches the fixed duration and whether cumulative total duration of the valid vital sign data is greater than a threshold, whether to perform blood glucose assessment on the user.

For example, the fixed duration is 10 days. It is assumed that the valid vital sign data includes the first valid vital sign data and the second valid vital sign data, the first cumulative total duration of the first valid vital sign data is 60 hours, the second cumulative total duration of the second valid vital sign data is 40 hours, the first threshold is 56 hours, and the second threshold is 35 hours. When the duration of the first cycle reaches 10 days, the electronic device 100 may perform blood glucose assessment on the user; or when the duration of the first cycle reaches 10 days, 60 hours is greater than 40 hours, and 56 hours is greater than 35 hours, the electronic device 100 may perform blood glucose assessment on the user.

It should be noted that, if the duration of the first cycle is the fixed duration, but the cumulative total duration of the valid vital sign data is not greater than the threshold, the electronic device 100 cannot determine the blood glucose assessment result corresponding to the first cycle. In this case, the electronic device 100 ends the first cycle, and automatically starts a next cycle of the blood glucose assessment. Optionally, the electronic device 100 may display corresponding reminder information to the user, to remind that the blood glucose assessment result corresponding to the first cycle is not obtained.

Case 2: The duration of the first cycle is one in a preset duration range.

In this embodiment of this application, the preset duration range includes a minimum value and a maximum value.

Optionally, the electronic device 100 may determine, based on whether the duration of the first cycle is one in the preset duration range and whether cumulative total duration of the valid vital sign data is greater than a threshold, whether to perform blood glucose assessment on the user.

For example, it is assumed that the valid vital sign data includes the first valid vital sign data and the second valid vital sign data, the preset duration range is 7 to 14 days, the first cumulative total duration of the first valid vital sign data is 60 hours, the second cumulative total duration of the second valid vital sign data is 40 hours, the first threshold is 56 hours, and the second threshold is 35 hours. When the duration of the first cycle reaches 10 days (10 is greater than 7 and less than 14), 60 hours is greater than 40 hours, and 56 hours is greater than 35 hours, the electronic device 100 may perform blood glucose assessment on the user.

According to Case 2, this can avoid, based on the minimum value of the preset duration range, a case in which an amount of the valid vital sign data is extremely small due to excessively short duration of the first cycle; and this can avoid, based on the maximum value of the preset duration range, a case in which valid vital sign data collected in a previous stage of the cycle is invalid due to excessively long duration of the first cycle.

A specific value in the preset duration range is specifically the duration of the first cycle may be obtained based on the following process:
For example, the minimum value of the preset duration range is seven days, and a maximum value is fourteen days. When the electronic device 100 collects first vital sign data and second vital sign data of seven days, the electronic device 100 may determine first cumulative total duration and second cumulative total duration of the seven days. If the first cumulative total duration of the seven days is greater than the first threshold, and the second cumulative total duration of the seven days is greater than the second threshold, the electronic device 100 determines that the duration of the first cycle is seven days, and may perform blood glucose assessment based on first valid vital sign data and second valid vital sign data of the seven days. If the first cumulative total duration of the seven days is not greater than the first threshold, and/or the second cumulative total duration of the seven days is not greater than the second threshold, the electronic device 100 continues to collect vital sign data on an eighth day. After the vital sign data on the eighth day is collected, the electronic device 100 determines whether first cumulative total duration of eight days is greater than the first threshold, and whether second cumulative total duration of the eight days is greater than the second threshold. If determining that the first cumulative total duration of the eight days is greater than the first threshold, and the second cumulative total duration of the eight days is greater than the second threshold, the electronic device 100 determines that the duration of the first cycle is eight days, and may perform blood glucose assessment based on the first valid vital sign data and the second valid vital sign data of the eight days. If determining that the first cumulative total duration of the eight days is not greater than the first threshold, and/or the second cumulative total duration of the eight days is not greater than the second threshold, the electronic device 100 continues to collect vital sign data and determine the first cumulative total duration and the second cumulative total duration, until determining that the first cumulative total duration is greater than the first threshold and the second cumulative total duration is greater than the second threshold on a 14^{th} day or before the 14^{th} day, and performs blood glucose assessment on the user.

It should be noted that, if determining that the first cumulative total duration of 14 days is still not greater than the first threshold, and/or the second cumulative total duration of the 14 days is still not greater than the second threshold on the 14^{th} day, the electronic device 100 cannot determine the blood glucose assessment result corresponding to the first cycle. In this case, the electronic device 100 ends the first cycle, and automatically starts a next cycle of the blood glucose assessment. Optionally, the electronic device 100 may display corresponding reminder information to the user, to remind that the blood glucose assessment result corresponding to the first cycle is not obtained.

Case 3: The duration of the first cycle is neither preset fixed duration nor one in a preset duration range.

In this embodiment of this application, the duration of the first cycle may not belong to Case 1 and Case 2. In Case 3, the duration of the first cycle may be determined by using the valid vital sign data, to flexibly adjust the duration of the first cycle. For example, it is assumed that the valid vital sign data includes the first valid vital sign data and the second valid vital sign data, the first threshold is 56 hours, and the second threshold is 35 hours. When first cumulative total duration of first valid vital sign data collected by the electronic device 100 in first seven days is less than 56 hours, and/or second cumulative total duration of second valid vital sign data collected in the first seven days is less than 35 hours, and first cumulative total duration of first valid vital sign data collected in first eight days is greater than 56 hours, and second cumulative total duration of second valid vital sign data collected in the first eight days is greater than 35 hours, the duration of the first cycle is eight days.

When first cumulative total duration of first valid vital sign data collected by the electronic device 100 in first nine days is less than 56 hours, and/or second cumulative total duration of second valid vital sign data collected in the first nine days is less than 35 hours, and first cumulative total duration of first valid vital sign data collected in first 10 days is greater than 56 hours, and second cumulative total duration of second valid vital sign data collected in the first 10 days is greater than 35 hours, the duration of the first cycle is 10 days.

S403: The electronic device 100 displays the blood glucose assessment result corresponding to the first cycle.

In a first possible implementation, the electronic device 100 may actively display the blood glucose assessment result corresponding to the first cycle. To be specific, after obtaining the blood glucose assessment result corresponding to the first cycle, the electronic device 100 directly displays the blood glucose assessment result.

In a second possible implementation, the electronic device 100 may display, in response to an operation of viewing the blood glucose assessment result by the user, the blood glucose assessment result corresponding to the first cycle. To be specific, the user may actively initiate the operation of viewing the blood glucose assessment result, and the electronic device 100 displays, in response to the operation, the obtained blood glucose assessment result corresponding to the first cycle.

In a third possible implementation, the electronic device 100 displays a view reminder pop-up window for the blood glucose assessment result; and in response to a second operation on the view reminder pop-up window, displays the blood glucose assessment result corresponding to the first cycle. To be specific, after obtaining the blood glucose assessment result corresponding to the first cycle, the electronic device 100 first stores the blood glucose assessment result corresponding to the first cycle. In addition, the electronic device 100 displays the view reminder pop-up window for the blood glucose assessment result, to remind the user "the blood glucose assessment result is generated and can be viewed". If the user initiates the second operation by using the view reminder pop-up window, the electronic device 100 displays the blood glucose assessment result corresponding to the first cycle.

In the third possible implementation, for example, FIG. 5 is a diagram of some blood glucose assessment result interfaces according to an embodiment of this application. After obtaining the blood glucose assessment result corresponding to the first cycle, the electronic device 100 displays an interface 510. The interface 510 may be actively popped up and displayed in a form of a pop-up window. The interface 510 includes view reminder information of the blood glucose assessment result (namely, it is found that your blood glucose assessment result from December 7 to December 14 is generated), a "view details" control, and an "ignore" control. When receiving the operation 511 on the "view details" control, the electronic device 100 displays one of interfaces 520 to 540. The interfaces 520 to 540 each include a blood glucose assessment result and corresponding advice. The interface 520 shows a blood glucose assessment result indicating a low-risk level of hyperglycemia of the user from December 7 to December 14, and corresponding advice on a low risk (namely, no significant risk of hyperglycemia is found, and you are advised to balance calorie intake with physical activity expenditure and ensure sufficient sleep time). The interface 530 shows a blood glucose assessment result indicating a medium-risk level of hyperglycemia of the user from December 7 to December 14, and corresponding advice on a medium risk (namely, there is a potential risk of hyperglycemia, and you are advised to wear a continuous blood glucose monitoring device to learn of a fasting blood glucose level and a postprandial blood glucose level in two hours after eating). The interface 540 shows a blood glucose assessment result indicating a high-risk level of hyperglycemia of the user from December 7 to December 14, and corresponding advice on a high risk (namely, there is a high risk of hyperglycemia, and if you want to pay further attention to a blood glucose health status, go to a hospital for examination and assessment).

It should be additionally noted that when the user is currently busy, the user may initiate an operation on the "ignore" control, the electronic device 100 does not display any one of the interfaces 520 to 540 after receiving the operation on the "ignore" control, and the user can subsequently view, in a blood glucose assessment record, a blood glucose assessment result that is not displayed and corresponding advice. The blood glucose assessment record is illustrated by subsequent FIG. 6E.

It may be understood that, if the electronic device 100 uses the first possible implementation, any one of the interface 520 to the interface 540 may be directly displayed;
if the electronic device 100 uses the second possible implementation, any one of the interface 520 to the interface 540 may be displayed after receiving a view operation of the user. In addition, when the second possible implementation is implemented, if the view operation of the user is initiated before the blood glucose assessment result corresponding to the first cycle is obtained, the electronic device 100 does not obtain the blood glucose assessment result corresponding to the first cycle, and may display an interface 550. The interface 550 indicates that effective wear duration of the user from December 7 to December 14 is insufficient, and no blood glucose assessment result is temporarily available.

S404: The electronic device 100 automatically starts a next cycle of the blood glucose assessment.

In this embodiment of this application, after obtaining the blood glucose assessment result corresponding to the first cycle, the electronic device 100 may automatically start the next cycle of the blood glucose assessment, to obtain a blood glucose assessment result corresponding to the next cycle. Based on this, the electronic device 100 may cyclically perform S401 to S404, to sequentially obtain blood glucose assessment results corresponding to a plurality of cycles.

For example, a manner of automatically starting the next cycle may be automatically starting the next cycle immediately, or automatically starting the next cycle after a preset time period.

It can be learned that according to this embodiment of this application, in a process in which the user wears the electronic device 100, blood glucose assessment may be periodically and automatically performed on the user, and in the process, the user does not need to actively enter a large amount of related information. This helps reduce operation complexity for the user.

In addition, this manner is a noninvasive blood glucose assessment method. Before a blood glucose assessment result is obtained, the user needs neither to always maintain a static state nor to calibrate the blood glucose assessment result. This helps improve user experience. When the risk level of hyperglycemia of the user is the medium or high risk, or the user is among the pre-diabetic population or the diabetic population, this manner can be used to warn the user in a timely manner, so that the user can learn of a potential risk in a timely manner and manage blood glucose health as soon as possible.

Optionally, based on the foregoing blood glucose assessment method, after the first cycle of the blood glucose assessment starts, the electronic device 100 may display related content of the blood glucose assessment. The related content of the blood glucose assessment includes content that the user expects to view, and content that the user needs to be reminded of in addition to the content that the user expects to view. Display of the content that the user expects to view may be triggered by an operation actively initiated by the user.

For example, the content that the user expects to view includes but is not limited to one or more of the following: blood glucose assessment progress, a wear status, the blood glucose assessment record, and the like.

### 1. Blood glucose assessment progress and/or daily wear status

In a possible implementation, the electronic device 100 may receive a first operation of the user in the first cycle; and in response to the first operation, display blood glucose assessment progress corresponding to the first cycle and/or a daily wear status of the wearable device, where the daily wear status indicates whether daily effective wear duration for the wearable device in the first cycle meets a standard.

The daily effective wear duration means duration of valid vital sign data in vital sign data collected by the electronic device 100 on each day when the user wears the electronic device 100. When the duration of the valid vital sign data collected on each day meets the standard, the daily effective wear duration meets the standard. When the duration of the valid vital sign data collected on each day does not meet the standard, the daily effective wear duration does not meet the standard.

Optionally, if the valid vital sign data includes the first valid vital sign data and the second valid vital sign data, when both the first vital sign data and the second vital sign data that are collected on each day meet the standard, the daily effective wear duration meets the standard. When either of the first vital sign data and the second vital sign data that are collected on each day does not meet the standard, the daily effective wear duration does not meet the standard.

For example, FIG. 6A to FIG. 6C are diagrams of some main interfaces of blood glucose health research displayed by an electronic device 100 according to an embodiment of this application. The main interfaces of the blood glucose health research shown in FIG. 6A to FIG. 6C includes the blood glucose assessment progress, the daily wear status, and a wear description. The daily wear status indicates whether the effective wear duration for which the user wears the electronic device 201 meets the standard (for example, a dark gray circle indicates that the effective wear duration meets the standard, and a light gray circle indicates that the effective wear duration is insufficient (that is, does not meet the standard)). The electronic device 100 may receive an operation performed by the user on the wear description, and further display a related description of wearing the electronic device 100 by the user (for example, completing one round of the blood glucose assessment takes at least 7 days and at most 14 days, and a watch needs to be worn in the daytime and at the night).

Specifically, FIG. 6A is a diagram of an example of a main interface of the blood glucose health research. The main interface of the blood glucose health research is displayed by the electronic device 100, and the display of the main interface may be triggered by an operation initiated by the user when the first cycle starts (when the assessment just starts). Specifically, blood glucose assessment progress 601 may be 0%, a daily wear status 602 does not include a status of wearing the electronic device 100 by the user, the daily wear status 602 may further indicate that the user wears the electronic device 100 today (the seventh day in a month).

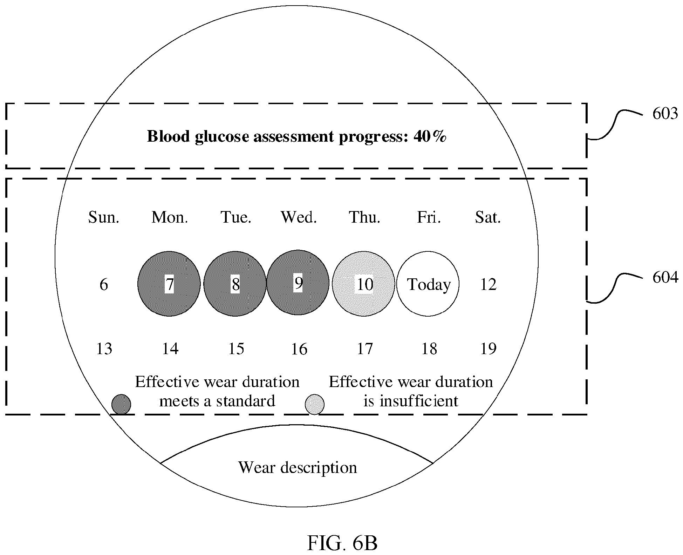

FIG. 6B is a diagram of an example of another main interface of the blood glucose health research. The main interface of the blood glucose health research is displayed by the electronic device 100, and the display of the main interface may be triggered by an operation initiated by the user after the first cycle starts and before the first cycle ends. For example, the first cycle starts on the seventh day in the month, and the duration of the first cycle is one of 7 to 14 days. The user may view the interface shown in FIG. 6B on the 11th day in the month (namely, a fifth day after the first cycle starts). Specifically, blood glucose assessment progress 603 may be 40%, and a daily wear status 604 includes daily wear statuses of first four days in the first cycle. Daily wear statuses on a first day to a third day in the first cycle (the seventh day to the ninth day in the month) indicate that effective wear duration meets the standard. A daily wear status on a fourth day in the first cycle (the 10^{th} day in the month) indicates that effective wear duration does not meet the standard.

FIG. 6C is a diagram of an example of still another main interface of the blood glucose health research. The main interface of the blood glucose health research is displayed by the electronic device 100, and the display of the main interface may be triggered by an operation initiated by the user after the first cycle starts and before the first cycle ends. For example, the first cycle starts on the seventh day in the month, and the duration of the first cycle is one of 7 to 14 days. The user may view the interface shown in FIG. 6C on the 19^{th} day in the month (namely, a 13^{th} day after the first cycle starts). Specifically, blood glucose assessment progress 605 may be 70%, and a daily wear status 606 includes daily wear statuses of first 12 days in the first cycle. Daily wear statuses on a first day to a third day, a fifth day, a 10^{th} day, and a 11^{th} day in the first cycle (namely, the seventh to the ninth, the 11^{th}, the 16^{th}, and the 17^{th} in the month) indicate that effective wear duration meets the standard. Daily wear statuses on a fourth day, a sixth day to a ninth day, and a 12^{th} day in the first cycle (namely, the 10^{th} day, the 12^{th} day to the 15^{th} day, and the 18^{th} day in the month) indicate that effective wear duration does not meet the standard.

Optionally, the user may further operate the daily wear statuses (namely, the dark gray circle/light gray circle) in FIG. 6A to FIG. 6C, to view specific daily effective wear duration, ineffective wear duration, and the like.

It may be understood that, the display forms of the blood glucose assessment progress and the daily wear status are merely examples, and may alternatively be displayed in another form during specific implementation. For example, the blood glucose assessment progress is displayed in a form of a graphical progress bar. For another example, if the vital sign data collected on each day includes the first vital sign data collected in the first time period and the second vital sign data collected in the second time period, the daily wear status may be displayed in a manner shown in FIG. 6D.

Daily wear status on a first day in the first cycle is used as an example. If the daily wear status is shown in (a) in FIG. 6D, 610 indicates that a wear status of a first time period on the first day meets the standard, and 611 indicates that a wear status of a second time period on the first day meets the standard. If the daily wear status is shown in (b) in FIG. 6D, 612 indicates that a wear status of a first time period on the first day meets the standard, and 613 indicates that a wear status of a second time period on the first day does not meet the standard. If the daily wear status is shown in (c) in FIG. 6D, 614 indicates that a wear status of a first time period on the first day does not meet the standard, and 615 indicates that a wear status of a second time period on the first day meets the standard. If the daily wear status is shown in (d) in FIG. 6D, 616 indicates that a wear status of a first time period on the first day does not meet the standard, and 617 indicates that a wear status of a second time period on the first day does not meet the standard.

In addition, the blood glucose assessment progress and the daily wear status may be displayed in a same interface (as shown in FIG. 6A to FIG. 6C) or in different interfaces. This is not limited in this application.

### 2. Blood glucose assessment record

In a possible implementation, the electronic device 100 may receive the first operation of the user in the first cycle; and in response to the first operation, display a blood glucose assessment record corresponding to the first cycle. The blood glucose assessment record may be displayed in an interface that is the same as that of the blood glucose assessment progress and/or the daily wear status, or may be displayed in an interface that is different from that of the blood glucose assessment progress and the daily wear status (for example, the user performs a slide-down operation in the interfaces shown in FIG. 6A to FIG. 6C, to trigger display of a blood glucose assessment record interface). This is not limited in this application. The blood glucose assessment record includes a blood glucose assessment result corresponding to a cycle before the first cycle.

For example, the blood glucose assessment record displayed by the electronic device 100 includes blood glucose assessment results corresponding to all cycles before the first cycle, or blood glucose assessment results corresponding to several latest cycles (for example, a preset quantity of cycles) before the first cycle.

FIG. 6E is a diagram of an example of a display interface of a blood glucose assessment record. For example, the display interface of the blood glucose assessment record includes three latest blood glucose assessment results, and the three blood glucose assessment results are arranged in descending order of time. A blood glucose assessment result of November 30 to December 6 shown in 621 indicates that the risk level of hyperglycemia of the user is a low risk. A blood glucose assessment result of November 23 to November 29 shown in 622 indicates that the risk level of hyperglycemia of the user is a low risk. A blood glucose assessment result of November 16 to November 22 shown in 623 indicates that the risk level of hyperglycemia of the user is a low risk.

It should be noted that if a quantity of all the cycles before the first cycle exceeds the preset quantity of cycles, the user may further view a remaining blood glucose assessment record on the electronic device 200. In addition, FIG. 6E shown in this application briefly displays only a plurality of blood glucose assessment results. The user may still view a detailed blood glucose assessment result and/or corresponding advice by operating each displayed blood glucose assessment result, for example, as shown in interfaces 520 to 540 in FIG. 5.

It can be learned that in a blood glucose assessment process, the user may actively initiate an operation to view the blood glucose assessment progress and/or the daily wear status and/or the blood glucose assessment record, learn of assessment progress of a current cycle in a timely manner by viewing the blood glucose assessment progress, learn whether the daily effective wear duration of the user meets the standard by viewing the daily wear status, and learn of the blood glucose assessment result corresponding to the cycle before the first cycle by viewing the blood glucose assessment record.

For example, the content that the user needs to be reminded of includes but is not limited to one or more of the following: a wear status reminder, a reminder of viewing the blood glucose assessment result, a blood glucose assessment progress reminder, a high risk reminder, and the like.

It may be understood that the content that the user needs to be reminded of may be displayed in a form of a pop-up window after the user receives the first operation. In addition, after displaying the content that the user needs to be reminded of, the electronic device may further display the content that the user expects to view (the blood glucose assessment progress and/or the daily wear status of the wearable device).

Alternatively, the content that the user needs to be reminded of may be content that is actively displayed in a form of a pop-up window, without being triggered by the first operation or another operation.

### 1. Wear status reminder

In a possible implementation, the electronic device 100 determines a wear status of a previous day, and if the wear status of the previous day indicates that the effective wear duration for the wearable device on one day before receiving of the first operation does not meet the standard, displays the wear status of the previous day.

FIG. 7A is a diagram of an example of a reminder interface of a wear status of a previous day. The reminder interface of the wear status of the previous day includes the wear status 710 of the previous day and an "OK" control 711. The wear status 710 of the previous day indicates that the effective wear duration of the previous day is insufficient, and then the wear status of the previous day may further remind the user to wear the wearable device normally and maintain the resting/sleep state as much as possible. The "OK" control 711 may be configured to: receive an operation of the user, and display, after receiving the operation of the user, the interfaces shown in FIG. 6A to FIG. 6C.

Optionally, the wear status of the previous day may further indicate that effective wear duration of a first time period on the previous day does not meet the standard, and/or effective wear duration of a second time period on the previous day does not meet the standard.

Based on this manner, when the wear status of the previous day indicates that the effective wear duration does not meet the standard, the user may be reminded in a timely manner, so that the user can adjust a wear habit as soon as possible.

### 2. Reminder of viewing the blood glucose assessment result

In a possible implementation, the electronic device 100 determines whether a blood glucose assessment result of a previous cycle has been displayed to the user; and if the blood glucose assessment result of the previous cycle has not been displayed to the user, displays the blood glucose assessment result of the previous cycle.

In this application, after the electronic device 100 obtains the blood glucose assessment result of the previous cycle, if the electronic device 100 does not actively display the blood glucose assessment result of the previous cycle, the electronic device 100 may actively display, to the user, the blood glucose assessment result of the previous cycle that is not displayed. Alternatively, when displaying the interface 510 shown in FIG. 5 in the previous cycle, the user taps the "ignore" control, and does not view the blood glucose assessment result of the previous cycle. In this case, the electronic device 100 may actively display, to the user, the blood glucose assessment result of the previous cycle that has not been displayed.

FIG. 7B is a diagram of an example of a reminder interface of a blood glucose assessment result. The reminder interface of the blood glucose assessment result includes a blood glucose assessment result 712 of the previous cycle and an "OK" control 713. The blood glucose assessment result 712 of the previous cycle indicates a medium-risk level of hyperglycemia of the user from December 7 to December 14, and corresponding advice on a medium risk (namely, there is a potential risk of hyperglycemia, and you are advised to wear a continuous blood glucose monitoring device to learn of a fasting blood glucose level and a postprandial blood glucose level in two hours after eating). The "OK" control 713 may be configured to receive an operation of the user. After receiving the operation of the user, the electronic device 100 may display the interfaces (namely, the blood glucose assessment record and daily wear status) shown in FIG. 6A to FIG. 6C, and may directly turn off a screen to enter the sleep state.

Based on this manner, when the user does not view the blood glucose assessment result of the previous cycle, the blood glucose assessment result of the previous cycle may be displayed to the user in a timely manner.

### 3. Blood glucose assessment progress reminder

In a possible implementation, when the blood glucose assessment progress corresponding to the first cycle reaches preset progress, the electronic device 100 displays the blood glucose assessment progress corresponding to the first cycle and to-be-worn effective duration, where the to-be-worn effective duration indicates duration for which the user still needs to effectively wear the wearable device.

In this application, when the blood glucose assessment progress corresponding to the first cycle reaches the preset progress, the electronic device 100 may actively display current blood glucose assessment progress to the user and display effective duration (namely, the to-be-worn effective duration) for which the user still needs to wear the electronic device 100.

Optionally, the electronic device 100 may further display a quantity of days for which the user still needs to wear the electronic device 100, effective duration of already wearing the electronic device 100, a quantity of days for which the user persists in wearing the electronic device 100 from the first cycle to a current moment, and the like. This is not limited in this application. Alternatively, the electronic device 100 may further display total effective wear duration for which the electronic device 100 should be worn in the first cycle, a preset duration range to which the duration of the first cycle belongs, and the like.

FIG. 7C is a diagram of an example of a reminder interface of blood glucose assessment progress. The reminder interface of the blood glucose assessment progress includes blood glucose assessment progress 714 and wear progress 715. The blood glucose assessment progress 714 is 50%, and the wear progress 715 is "you have persisted for four days, the effective wear duration is 35 hours, and you can obtain the blood glucose assessment result once wearing the electronic device for another 35 hours". The "35 hours" in "wearing the electronic device for another 35 hours" is the to-be-worn effective duration. The "four days" in "you have persisted for four days" is the quantity of days for which the user persists in wearing the electronic device 100 from the first cycle to the current moment. The 35 hours in "the effective wear duration is 35 hours" is the effective duration of already wearing the electronic device 100.

FIG. 7D is a diagram of an example of another reminder interface of blood glucose assessment progress. The reminder interface of blood glucose assessment progress includes blood glucose assessment progress 716 and wear progress 717. The blood glucose assessment progress 716 is 50%, and the wear progress 717 is "persist in effectively wearing the electronic device for 70 hours in 7 to 14 days, to obtain the blood glucose assessment result. * the effective wear duration is 35 hours * the ineffective wear duration is 35 hours". The "35 hours" in "the ineffective wear duration is 35 hours" is the to-be-worn effective duration. The "7 to 14 days" is the preset duration range. The "70 hours" in "persisting in wearing the electronic device for 70 hours" is total effective wear duration. The "35 hours" in "the effective wear duration is 35 hours" is the effective duration of already wearing the electronic device 100.

Based on this manner, in the blood glucose assessment process, the blood glucose assessment progress and the to-be-worn effective duration may be actively displayed to the user, to encourage the user to persist in wearing the electronic device 100.

### 4. High Risk reminder

In a possible implementation, before displaying the blood glucose assessment result corresponding to the first cycle, if the blood glucose assessment result corresponding to the first cycle indicates that a risk level of hyperglycemia is a high risk and/or indicates that the user is among a diabetic population, the electronic device 100 displays hyperglycemia high-risk reminder information and/or hyperglycemia health reminder information.

In this application, if the electronic device 100 is triggered by the user to display the blood glucose assessment result corresponding to the first cycle, the electronic device 100 does not display the blood glucose assessment result corresponding to the first cycle when no trigger operation of the user is received. When the blood glucose assessment result indicates that the risk level of hyperglycemia is a high risk, it is likely to miss time for diagnosis and treatment. Based on this, before displaying the blood glucose assessment result corresponding to the first cycle, the electronic device may directly display the hyperglycemia high-risk reminder information and/or the hyperglycemia health reminder information.

FIG. 7E is a diagram of an example of a reminder interface of a high risk. The reminder interface of the high risk includes the high risk reminder. The high risk reminder is "it is found that your blood glucose assessment result from October 20 to October 25 is the "high risk"; seek medical treatment in time if feeling uncomfortable; tap to view details". The user may tap the interface shown in FIG. 7E, to learn of specific information about the blood glucose assessment result corresponding to the first cycle. The specific information of the blood glucose assessment result corresponding to the first cycle is not described in this application, and may include effective wear duration of the first cycle, corresponding advice, and the like.

Based on this manner, when it is determined that the risk level of hyperglycemia is the high risk or the user is among the diabetic population, the user may be notified in a timely manner, to avoid missing a diagnosis and treatment opportunity.

It should be noted that when two or more of the four types of content meet a display trigger condition, the electronic device 100 may sequentially display the two or more types of the content based on a specified priority. For example, if a priority of the reminder of viewing the blood glucose assessment result is higher than a priority of the blood glucose assessment progress reminder, the reminder of viewing the blood glucose assessment result is displayed first, and then the blood glucose assessment progress reminder is displayed.

It may be understood that the content in FIG. 5, FIG. 6A to FIG. 6E, and FIG. 7A to FIG. 7E may be further displayed on the electronic device 200.

FIG. 8A is a diagram of an example of a main interface of displaying blood glucose health research by an electronic device 100. The main interface of the blood glucose health research includes blood glucose assessment progress and daily wear status 810, and a blood glucose assessment record 811.

For the blood glucose assessment progress and daily wear status 810, refer to the detailed description in FIG. 6B.

The blood glucose assessment record includes three latest blood glucose assessment results and a "more" control 812, and the three blood glucose assessment results are arranged in descending order of time. For the three blood glucose assessment results, refer to the detailed description in FIG. 6E. For example, one latest blood glucose assessment result (for example, 813) may be displayed in a detailed form, and remaining two blood glucose assessment results (for example, 814) may be displayed in a brief form. The user can operate to display the blood glucose assessment record in the brief form, to view the detailed blood glucose assessment record shown in 813. For example, the user may operate the "more" control 812 to view all blood glucose assessment records.

FIG. 8B is a diagram of a main interface of a blood glucose assessment record displayed after the user operates the "more" control 912. The main interface of the blood glucose assessment record includes a blood glucose assessment record of December 814 and a blood glucose assessment record of November 815. The blood glucose assessment record of December 814 shows a collapsed blood glucose assessment record. The blood glucose assessment record of November 815 shows an expanded blood glucose assessment record. The expanded blood glucose assessment records include four blood glucose assessment results in November. A view operation performed by the user on any one of the four blood glucose assessment results may be received, to display a blood glucose assessment result corresponding to the view operation and corresponding advice. For example, the view operation on a blood glucose assessment result of November 2 to November 8 is received, and a blood glucose assessment result shown in FIG. 8C is displayed. For content shown in FIG. 8C, refer to the description of the interface 520 in FIG. 5. Details are not described herein again.

Optionally, after obtaining the blood glucose assessment result corresponding to the first cycle, the user may further upload a result feedback for the blood glucose assessment result corresponding to the first cycle. For example, the result feedback may be used to update a blood glucose assessment model.

In a possible implementation, after displaying the blood glucose assessment result corresponding to the first cycle, the electronic device 100 obtains the result feedback for the blood glucose assessment result corresponding to the first cycle, where the result feedback indicates that the blood glucose assessment result corresponding to the first cycle is different from an actual blood glucose assessment result; and updates the blood glucose assessment model based on the result feedback, where the blood glucose assessment model is used to determine a blood glucose assessment result based on the valid vital sign data in the vital sign data.

The user may feed back whether the blood glucose assessment result corresponding to the first cycle is the same as the actual blood glucose assessment result (including same, different, and uncertain). When the blood glucose assessment result corresponding to the first cycle is different from the actual blood glucose assessment result, the blood glucose assessment model is updated based on the result feedback. For example, the blood glucose assessment result corresponding to the first cycle that is obtained by the electronic device 100 indicates that the risk level of hyperglycemia of the user is a high risk. However, the actual blood glucose assessment result measured by the user in a hospital indicates that the risk level of hyperglycemia of the user is the low risk, it indicates that the blood glucose assessment result corresponding to the first cycle is inaccurate, and the blood glucose assessment model may be updated based on the actual blood glucose assessment result corresponding to the result feedback. Optionally, such result feedback may be displayed on the electronic device 100 or the electronic device 200.

For example, FIG. 9A to FIG. 9B are a diagram of interfaces of displaying a result feedback on the electronic device 200. As shown in FIG. 9A, a blood glucose assessment record displayed on the electronic device 200 may further include a "result feedback" control 901. The user may operate the "result feedback" control 901 to display a main interface of the result feedback shown in FIG. 9B. The main interface of the result feedback includes a "select" control 902 and a "submit" 903. The user may first operate the "select" control 902 to determine whether a latest blood glucose assessment result is consistent with an actual blood glucose assessment result, and then operate the "submit" 903 to submit the result feedback.

Optionally, the user may further upload data (for example, an examination report) related to the blood glucose assessment, to provide a better blood glucose management service for the user or update the blood glucose assessment model. For example, the data related to the blood glucose assessment is a related report of blood glucose detection in a hospital, and an exercise plan, a diet plan, a medication plan, or the like for reducing blood glucose may be formulated for the user based on the obtained related data (the data is accurate data). In addition, a blood glucose change trend of the user is predicted based on an execution degree of the exercise plan, the diet plan, and the medication plan, and displayed to the user.

For example, FIG. 9C to FIG. 9D are a diagram of interfaces of displaying and uploading data related to blood glucose assessment on an electronic device 200. As shown in FIG. 9A, the blood glucose assessment record displayed on the electronic device 200 may further include an "upload" control 904 of a report related to the blood glucose assessment. The user may operate the "upload" control 904 to display a main interface of report uploading shown in FIG. 9D. The main interface of report uploading includes a report description area 905, a report format selection area 906, and a report uploading area 907. The user may describe a report name and examination time in the report description area 905, and select a format of a to-be-uploaded file in the report format selection area 906 (the file format includes but is not limited to one or more of a picture and a document). After selecting at least one file format, the user may upload a report in a corresponding file format in the report uploading area 907.

An "embodiment" mentioned in this specification indicates that a particular feature, structure, or characteristic described with reference to this embodiment may be included in at least one embodiment of this application. The phrase shown in various locations in this specification may not necessarily mean a same embodiment, and is not an independent or optional embodiment exclusive from another embodiment. It is explicitly and implicitly understood by a person skilled in the art that embodiments described in this specification may be combined with another embodiment.

Terminologies such as "component", "module", and "system" used in this specification are used to indicate computer-related entities, hardware, firmware, combinations of hardware and software, software, or software being executed. For example, a component may be, but is not limited to, a process that runs on a processor, a processor, an object, an executable file, an execution thread, a program, and/or a computer. As illustrated by using figures, both a computing device and an application that runs on the computing device may be components. One or more components may reside within a process and/or a thread of execution, and a component may be located on one computer and/or distributed between two or more computers. In addition, these components may be executed from various computer-readable media that store various data structures. For example, the components may communicate by using a local and/or remote process and based on, for example, a signal having one or more data packets (for example, data from two components interacting with another component in a local system, a distributed system, and/or across a network like the internet interacting with other systems by using the signal).

In the foregoing embodiments, the description of each embodiment has respective focuses. For a part that is not described in detail in an embodiment, refer to related descriptions in other embodiments.

It should be noted that, for brief description, the foregoing method embodiments are represented as a series of actions. However, a person skilled in the art should appreciate that this application is not limited to the described order of the actions, because according to this application, some steps may be performed in other orders or simultaneously. It should be further appreciated by a person skilled in the art that embodiments described in this specification all belong to example embodiments, and the involved actions and modules are not necessarily required by this application.

In the several embodiments provided in this application, it should be understood that the disclosed apparatus may be implemented in other manners. For example, the described apparatus embodiment is merely an example. For example, division into the units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic or other forms.

The foregoing units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

In addition, functional units in embodiments of this application may be integrated into one processing unit, or each of the units may exist alone physically, or two or more units are integrated into one unit. The integrated unit may be implemented in a form of hardware, or may be implemented in a form of a software functional unit.

When the foregoing integrated unit is implemented in the form of a software functional unit and sold or used as an independent product, the integrated unit may be stored in a computer-readable storage medium. Based on such an understanding, the technical solutions of this application essentially, or the part contributing to the conventional technology, or all or some of the technical solutions may be implemented in the form of a software product. The computer software product is stored in a storage medium and includes several instructions for instructing a computer device (which may be a personal computer, a server, or a network device, and may be specifically a processor in the computer device) to perform all or some of the steps of the methods described in embodiments of this application. The foregoing storage medium includes any medium that can store program code, such as a USB flash drive, a removable hard disk, a magnetic disk, an optical disc, a read-only memory (Read-Only Memory, ROM for short), or a random access memory (Random Access Memory, RAM for short).

The foregoing embodiments are merely intended for describing the technical solutions of this application other than limiting this application. Although this application is described in detail with reference to the foregoing embodiments, a person of ordinary skill in the art should understand that they may still make modifications to the technical solutions described in the foregoing embodiments or make equivalent replacements to some technical features thereof, without departing from the spirit and scope of the technical solutions of embodiments of this application.

## Claims

1. A blood glucose assessment method, applied to a wearable device, wherein the method comprises:
collecting vital sign data of a user in a first cycle of blood glucose assessment;
performing blood glucose assessment on the user based on valid vital sign data in the vital sign data, to obtain a blood glucose assessment result corresponding to the first cycle;
displaying the blood glucose assessment result corresponding to the first cycle; and
starting a next cycle of the blood glucose assessment.

2. The method according to claim 1, wherein the vital sign data comprises first vital sign data collected in a first time period and second vital sign data collected in a second time period, the first time period and the second time period are two time periods on a same day, and the method further comprises:
determining first cumulative total duration and second cumulative total duration, wherein the first cumulative total duration is cumulative total duration for collecting first valid vital sign data in the first cycle, and the second cumulative total duration is cumulative total duration for collecting second valid vital sign data in the first cycle, the first valid vital sign data is valid vital sign data in the first vital sign data, and the second valid vital sign data is valid vital sign data in the second vital sign data; and
the performing blood glucose assessment on the user based on valid vital sign data in the vital sign data comprises:
if the first cumulative total duration is greater than a first threshold, and the second cumulative total duration is greater than a second threshold, performing blood glucose assessment on the user based on the first valid vital sign data in the first vital sign data and the second valid vital sign data in the second vital sign data.

3. The method according to claim 2, wherein duration of the first cycle is one in a preset duration range.

4. The method according to claim 2 or 3, wherein the method further comprises:
receiving a first operation of the user in the first cycle; and
in response to the first operation, displaying blood glucose assessment progress corresponding to the first cycle and/or a daily wear status of the wearable device, wherein the daily wear status indicates whether daily effective wear duration for the wearable device in the first cycle meets a standard.

5. The method according to claim 4, wherein before the displaying blood glucose assessment progress corresponding to the first cycle and/or a daily wear status of the wearable device, the method further comprises:
determining a wear status of a previous day, wherein the wear status of the previous day indicates whether effective wear duration for the wearable device on one day before receiving of the first operation meets the standard; and
if the wear status of the previous day indicates that the effective wear duration for the wearable device on one day before receiving of the first operation does not meet the standard, displaying the wear status of the previous day.

6. The method according to claim 4, wherein after the receiving a first operation of the user in the first cycle, and before the displaying blood glucose assessment progress corresponding to the first cycle and/or a daily wear status of the wearable device, the method further comprises:
determining whether a blood glucose assessment result of a previous cycle has been displayed to the user; and
if the blood glucose assessment result of the previous cycle has not been displayed to the user, displaying the blood glucose assessment result of the previous cycle.

7. The method according to any one of claims 2 to 6, wherein the method further comprises:
when the blood glucose assessment progress corresponding to the first cycle reaches preset progress, displaying the blood glucose assessment progress corresponding to the first cycle and to-be-worn effective duration, wherein the to-be-worn effective duration indicates duration for which the user still needs to effectively wear the wearable device.

8. The method according to any one of claims 1 to 7, wherein after the displaying the blood glucose assessment result corresponding to the first cycle, the method further comprises:
obtaining a result feedback for the blood glucose assessment result corresponding to the first cycle, wherein the result feedback indicates that the blood glucose assessment result corresponding to the first cycle is different from an actual blood glucose assessment result; and
updating a blood glucose assessment model based on the result feedback, wherein the blood glucose assessment model is used to determine a blood glucose assessment result based on the valid vital sign data in the vital sign data.

9. The method according to any one of claims 1 to 8, wherein before the displaying the blood glucose assessment result corresponding to the first cycle, the method further comprises:
if the blood glucose assessment result corresponding to the first cycle indicates that a risk level of hyperglycemia is a high risk and/or indicates that the user is among a diabetic population, displaying hyperglycemia high-risk reminder information and/or hyperglycemia health reminder information.

10. The method according to any one of claims 1 to 9, wherein the displaying the blood glucose assessment result corresponding to the first cycle comprises:
displaying a reminder pop-up window for the blood glucose assessment result; and
in response to a second operation on the view reminder pop-up window, displaying the blood glucose assessment result corresponding to the first cycle.

11. An electronic device, wherein the electronic device comprises one or more processors and one or more memories, the one or more memories are coupled to the one or more processors, the one or more memories are configured to store computer program code, the computer program code comprises computer instructions, and when the one or more processors execute the computer instructions, the electronic device is enabled to perform the method according to any one of claims 1 to 10.

12. A computer-readable storage medium, comprising computer instructions, wherein when the computer instructions are run on an electronic device, the electronic device is enabled to perform the method according to any one of claims 1 to 10.
